# EUROPEAN PATENT SPECIFICATION

(11) **EP 1 531 934 B1**
(45) Date of publication and mention of the grant of the patent: **24.04.2013**
(21) Application number: 03792155.8
(22) Date of filing: 26.08.2003
(51) Int. Cl.: B01L 3/00, G01N 35/00, G01N 33/48

(54) **A TESTING DEVICE FOR TESTING OR ANALYSING FLUIDS AND A HOLDER AND A STORAGE CONTAINER FOR SUCH DEVICES**
PRÜFEINRICHTUNG ZUM TESTEN ODER ANALYSIEREN VON FLÜSSIGKEITEN, HALTER UND LAGERBEHÄLTER FÜR SOLCHE EINRICHTUNGEN
DISPOSITIF DE TEST POUR TESTER ET ANALYSER DES FLUIDES, SUPPORT ET CONTENANT DE STOCKAGE POUR LEDIT DISPOSITIF

(30) Priority: 26.08.2002 DK 200201254; 26.08.2002 US 405711 P; 19.05.2003 DK 200300751
(43) Date of publication of application: 25.05.2005
(73) Proprietor: Lattec I/S, 3400 Hillerød (DK)
(72) Inventor: JOCHUMSEN, Hans, Henrik, DK-3450 Allerod (DK); FREDERIKSEN, Niels, Stubager, DK-2720 Vanl se (DK); RASMUSSEN, Janus, Juul, DK-3200 Helsinge (DK); CARLSEN, Thomas, Nikolai, DK-1663 Copenhagen-V (DK)
(74) Representative: Plougmann & Vingtoft A/S
(86) International application number: PCT/DK2003/000560
(87) International publication number: WO 2004/018100

(56) References cited:
- US-A- 4 142 863
- US-A- 4 346 817
- US-A- 5 256 372
- US-A- 5 592 289
- US-A- 5 599 505
- US-A- 6 118 582
- US-A1- 2002 102 149
- US-B1- 6 287 783
- US-B1- 6 303 389

## Description

The present invention relates to a testing device for testing or analysing fluids, a holder or a system in accordance with the appended claims.

It is known to make qualitative or quantitative analytical determination of constituents of fluids, such as body fluids of humans or animals, for example milk, by using so-called test carriers. Test carriers are usually strip-, sheet- or plate-like members including porous or fibrous test layers with one or more chemical reactants or reagents. When a liquid sample to be tested is applied to the test carrier and comes into contact with the reagents therein a detectable signal, such as a colour change, is generated. Such signal can be evaluated visually or by means of a suitable testing or analysing apparatus. Usually, the test carrier (in the following also referred to as "test member") is mounted in a plastic holder or frame so that it can be handled properly in an analysing apparatus. Such known holders or frames are disclosed in i.a. EP-A-0 511 120, US-A-4,230,757, US-A-4,387,990, US-A-5,173,261, US-A-5,244,632, and US-A-5,258,163.

US 6,303,389 and US 6,118,582 disclose different types of testing devices having a holder for holding a test member. The holders have planar lower and upper surfaces which means that a plurality of testing devices can be stacked on top of each other.

Because the known holders or frames are usually made from two or more separate parts, which have to be interconnected when the test carrier or test member is mounted, mounting of the test members in the known frames or holders is relatively complicated and time consuming, and replacement of the test member so that the holder may be re-used, is normally not possible in practise.

Therefore, the present invention has for its object to provide a testing device and a holder or frame allowing a very simple and easy mounting of the test member in the holder or frame.

Thus, the present invention provides a testing device for testing or analysing fluids and comprising at least one strip-, sheet- or plate-like test member including analysis reagent and having opposite side surfaces surrounded by edge portions, and a separately produced holder having retaining means for receiving and retaining the test member in a predetermined relative position in the holder, said retaining means comprising an abutment surface engaging with one of said side surfaces of the test member, and projections, which are positioned and shaped so as to allow insertion of the test member into the holder by moving the test member into engagement with said abutment surface while engaging with opposite edge portions thereof.

The testing device according to the invention allows for a very simple and easy mounting of the test member in the holder by simply pushing the test member towards the abutment surface. While the test member is pushed into abutting engagement with the abutment surface the projections are engaging with opposite edge portions of the test member. Thus, the projections will either locally deform opposite edge portions of the test member and/or penetrate into the material of such edge portions, whereby the test member may be retained in the desired position in the holder. Because the inner side surface of the test member is in abutting engagement with abutment surface of the holder, the correct mutual position in the holder is secured. Also, after use the test member may rather easily be removed and replaced by a fresh one, so that the holder may be re-used, if desired.

In the present context the term "sheet- or plate-like test member" should comprise not only a test member made from two or more layers comprising i.a. porous materials and plastic films, laminates etc., but also test members in the form of strips or rods, which could have been made by cutting a sheet or plate into such strips or rods, and test members having varying thicknesses along at least one dimension.

Preferably, the projections are tooth-shaped with pointed ends, whereby the projections can better bite or penetrate into the material of the test member edge portions. Alternatively or additionally, each of at least some of the projections may have a leading edge forming a ramp sloping towards a plane defined by the abutment surface so as to facilitate insertion of the test member into the holder. When the test member is inserted into the holder and is pushed or biased towards the abutment surface of the holder, the edge portions of the test member will first meet the inwardly sloping ramp-like leading edge of the projections. The sloping leading edges of the opposite projections cause a slight lateral compression of the opposite edge portions of the test member so that these edge portions may partly or completely pass the free ends of the projections, which may be pointed. Additionally, the leading edge of the projections may form a cutting edge so that the sloping cutting edge may at least partly cut into the edge portion of the test member, when the test member is moved into engagement with the abutment surface.

In order to prevent the test member from moving unintentionally out of engagement with the abutment surface the leading edge of at least some of the projections may be barbed, and/or the trailing edge of the projection may be shaped so that the free end of the projection functions like a barb. Thus, the trailing edge may slope in the same direction as the leading edge and define with the abutment surface an acute angle, which is smaller than the acute angle defined by the leading edge. Alternatively, each of at least some of the projections may have a trailing edge extending substantially parallel with and spaced from a plane defined by the abutment surface. It is also envisaged that the trailing edge or surface slope in a direction opposite to the direction of the leading edge. In this case the free pointed end of the projection may still function as a barb, and the trailing edge or surface may function as a supporting surface abuttingly engaging with the adjacent side surface opposite to the abutment surface provided that the said spacing substantially corresponds to the thickness of the sheet- or plate-like testing member. In order to allow test members of different thicknesses to be safely retained in a holder, the projections of the holder may be positioned so as to be differently spaced from the plane defined by the abutment surface. It is also envisaged that the projections may be formed by thermoplastic deformation of wall parts of the holder prior to or after insertion of the test member in the holder.

The terms "leading edge" and "trailing edge" as used herein should be understood as the edge coming first and the edge coming last, respectively, into engagement with the test member, when the test member is moved into the holder.

In one of the preferred embodiments the holder is a channel-shaped member having an inner bottom surface defining said abutment surface and opposite inner side surfaces from which projections extend in opposite directions. A holder of this type is suitable for use in connection with an elongated test member of the "lateral flow stick" type, in which the fluid to be tested is supplied at one end of the elongated test member.

In another preferred embodiment the holder is frame-shaped and defines an opening therein, and the abutment surface extends around and adjacent to said opening. Because projections may be positioned all way around the opening through which the test member is exposed it is possible to obtain a very exact position of the exposed side surface part in relation to the holder.

In order to facilitate storing of a stock of testing devices, the holder has upper and lower complementary surfaces so as to allow stacking of a plurality of testing devices on top of each other. These complementary surfaces are shaped so as to guide mutual displacement of stacked testing devices along a path of movement along one axis transversely to the longitudinal direction of the stack. As explained below, such features may be advantageous when the stacked testing devices are stored in a storage container or cassette from which they are dischargeable one by one.

The testing device may be used for testing of any type of liquid for which suitable test members of the type in question exist. In a presently preferred embodiment the testing device according to the invention is for use in colorimetric testing of milk.

According to a further aspect the present invention also relates to a holder according to claims 10-17 or 19, use in for a testing device as that described above, said holder comprising means for receiving and retaining a sheet- or plate-like test member, which has opposite side surfaces surrounded by edge portions, in a predetermined relative position in the holder, said retaining means comprising an abutment surface for engaging with one of said side surfaces of the test member and projections, which are positioned and shaped so as to allow insertion of the test member into the holder by moving the test member into engagement with said abutment surface while engaging with opposite edge portions thereof.

In principle, the holder according to the invention may be composed by two or more separate parts. In the preferred embodiment, however, the holder is formed integrally, for example by injection moulding or extrusion from plastic material.

The testing devices are suitably delivered to the user in a storage container or cassette, which may be connected to an analyser, and from which a testing device may be withdrawn one by one when needed. Even though the testing devices are usually fed into an automatic analyser, they may be discharged from the container or cassette manually. Usually the testing devices are disposed with after use. However, it is possible to remove the test member from the holder after use and to reuse the holder.

Thus, the invention also relates to such an elongated storage container or cassette for receiving a plurality of stacked testing devices of the type having at least one sheet- or plate-like test member including analysis reagent, and a holder receiving and retaining the test member in a predetermined relative position in the holder, said container comprising a movable support member for supporting a lower testing device in said stack, an upper abutment surface for engaging with an upper testing device in the stack, a discharge opening aligned with said upper testing device, so as to allow discharge of said upper testing device by displacing the same along said abutment surface. The movable support member may be biased towards the upper abutment surface, for example by means of a spring positioned in the lower part of the container or cassette. In the preferred embodiment the biasing means is part of the analyser, such as a piston or plunger moving trough an opening in the bottom part of the cassette.

In the latter case it is necessary to prevent that the movable support member moves in a direction away from the upper abutment surface, when the container or cassette is removed from the analyser and its biasing means. Therefore, the storage container preferably further comprises one-way means associated with the movable support member allowing the movable support member to move in a direction towards the upper abutment surface, only. These one-way means may, for example, comprise at least one succession of teeth, such as a rack or ratchet teeth, and at least one pawl member co-operating therewith.

Such a ratchet system may retain the support member in a plurality of positions being longitudinally spaced corresponding to the pitch of the succession of teeth. In order to increase the number of longitudinal positions, in which the support member may be retained for a given pitch of the rack or succession of teeth, the one-way means may comprise at least two pawl members, which are connected to the supporting member for co-operating with a succession of teeth formed on an inner side surface of the storage container, the free ends of the pawl members being spaced in the longitudinal direction of the container by a distance being different from a multiple of the pitch of the succession of teeth, preferably smaller than said pitch. Thus, if said spacing is for example half the pitch a number of the uniformly longitudinally spaced positions being the double of the number of teeth in the row or succession of teeth is obtainable.

According to a further aspect the invention also relates to a system comprising a cartridge for receiving, storing and unloading a plurality of stacked testing devices, wherein the cartridge comprises a housing defining an internal passage for said stack of testing devices and wherein the housing comprises: a lower charge opening for receiving said stack of testing devices, a movable support member for supporting a lower testing device in said stack, an upper abutment surface for engaging with an upper testing device in the stack, and an upper discharge opening, substantially aligned with said upper testing device, so as to allow discharge of said upper testing device by displacing the same along said abutment surface.

The housing of the cartridge may be assembled by two halves, together defining oppositely side surfaces, and a front and a back surface, and wherein the two halves are detachable or non-detachable assembled.

The cartridge further comprises a discharge opening which comprises guiding trails or incisions for guiding a testing device upon discharging.

The inside of the side surfaces of the cartridge may comprise guiding trails for guiding a stack of testing devices through the passage. The side surfaces further comprise at least one serrated track on the inside, forming one side of an internal one-way stair for a movable support member. The movable support member is movable in relation to the housing.

The one-way stair inside the cartridge allows the movable support member to move in a direction towards the upper abutment surface, only. The one-way means comprise at least one succession of teeth, such as a rack or ratchet teeth, and at least one pawl member co-operating therewith.

In a preferred embodiment, a cartridge comprises at least two pawl members, which are connected to the supporting member for co-operating with a succession of teeth formed on an inner side surface of the storage container, the free ends of the pawl members being spaced in the longitudinal direction of the container by a distance being different from a multiple of the pitch of the succession of teeth, preferably smaller than said pitch.

Such a cartridge further comprises a locking device in the vicinity of the discharge opening on at least one of the side surfaces, for preventing unintentional discharges of testing devices. The locking device comprises at least one flexible protrusion obstructing at least a part of said discharge opening.

The invention further relates to a system as described above and further comprising a load device for loading a stack of testing devices into a cartridge, the load device comprising: a base member, a first and a second column oppositely arranged and extending upwards from said base member, and being adapted to receive and hold one or more testing devices there between, and a lifting device for slidably lifting one of more testing devices along said columns. Each column comprises a groove for receiving and guiding an end of a test stick. The lifting device comprises a handle for manually sliding said lifting device along said columns. The lifting device is preferably automatically slid along said columns. The lifting device further comprises a support surface for supporting at least a part of the lower testing device in said stack of testing devices.

The movable support member is preferably arranged between said support surface of the lifting device and the lower most testing device in the stack.

In order to facilitate the guiding of the lifting device, the device comprises guiding means abutting a side portion of said columns so as to guide the device along the columns.

### BRIEF DESCRIPTION OF THE DRAWINGS

The invention will now be explained with reference to the drawings illustrating embodiments of the testing device, the holder and the storage container according to the invention, and wherein:
Fig. 1 is a top perspective view of a first embodiment of a holder according to the invention for a testing member,
Fig. 2 is a top perspective view of a first embodiment of the testing device according to the invention comprising the holder shown in Fig. 1,
Fig. 3 is a bottom perspective view of the testing device shown in Fig. 2,
Fig. 4 is a top perspective view of a second embodiment of a holder according to the invention,
Fig. 5 is a top perspective view of a second embodiment of the testing device according to the invention comprising the holder shown in Fig. 4,
Fig. 6 is a perspective view of a first embodiment of a storage container or cassette according to the invention for receiving a stack of testing devices as that shown in Figs. 2 and 3,
Fig. 7 is a perspective view of a second embodiment of a storage container or cassette according to the invention for receiving a stack of testing devices as that shown in Fig. 5,
Fig. 8 is a fragmentary perspective view of an upper part of the storage container or cassette of Fig. 7 shown in an enlarged scale,
Fig. 9 is a fragmentary perspective view of a lower part of the storage container or cassette of Fig. 7 shown in an enlarged scale, and
Fig. 10 is a fragmentary sectional view of the lower part of the storage container or cassette of Fig, 7 taken along a median plane of the container.
Fig. 11 is a top perspective view of a third embodiment of an empty holder according to the invention.
Fig. 12 is a top perspective view of a third embodiment loaded with a test stick.
Fig. 13 and 14 are a magnification of the central part of the holder shown in figure 11 and 12, illustrating two rib-shaped protrusions.
Fig. 15 is a top perspective of a second embodiment of an empty holder with a test stick above.
Fig. 16 shows two LS frames piled up.
Fig. 17 is a top perspective view of the fourth embodiment with a test stick above.
Fig. 18 is a top perspective view of the fourth embodiment loaded with a test stick.
Fig. 19 is a top perspective view of the third embodiment of an empty holder according to the invention.
Fig. 20 is a bottom perspective view of the fourth embodiment according to the invention, showing the point of injection and the marks from the ejection pins.
Fig. 21 is a profile view of the embodiments one to four, according to the invention.
Fig. 22 shows two frames piled up on top of each other.
Fig. 23 shows the drawings of the third embodiment.
Fig. 24 shows the drawings of the fourth embodiment.
Fig. 25 is a perspective view of a third embodiment of a storage container or cassette according to the invention for receiving a stack of testing devices as shown in Figs. 5, 11 and 12.
Fig. 26 is a perspective view of a fourth embodiment of a storage container or cassette according to the invention for receiving a stack of testing devices as shown in Fig. 2, 3, and 19.
Fig. 27 shows a cross-section of a cassette before assembly 27a and after assembly 27b.
Fig. 28 shows a magnification of the assembly points in picture 28, the left figure 28a is before assembly and the right figure 28b is after assembly.
Fig. 29 shows the energy directors on one of the halves of a cartridge.
Fig. 30 shows a cross-section of an assembled cartridge having a test slide inside.
Fig. 31 shows a magnification of the corner guides.
Fig. 32 shows the top part of a cartridge according to the fourth embodiment. Fig. 33 shows a cross-section of the resilient finger.
Fig. 34 shows a cross-section of a cartridge according to the fourth embodiment.
Fig. 35 shows an edge view of a cartridge.
Fig. 36 shows two embodiments of the movable bottom plate.
Fig. 37 shows a cartridge loaded with holders.
Fig. 38 is a magnification of the mounting of a movable bottom plate.
Fig. 39 shows a mounting tool for loading cartridges.
Fig. 40 shows holders piled-up in the mounting tool or loading device.
Fig. 41 shows a cartridge placed over the sticks and the two guiding legs of the mounting tool. Furthermore a lifting device is shown in this figure.
Fig. 42 shows the same as figure 41, with the lifting device in a raised position.

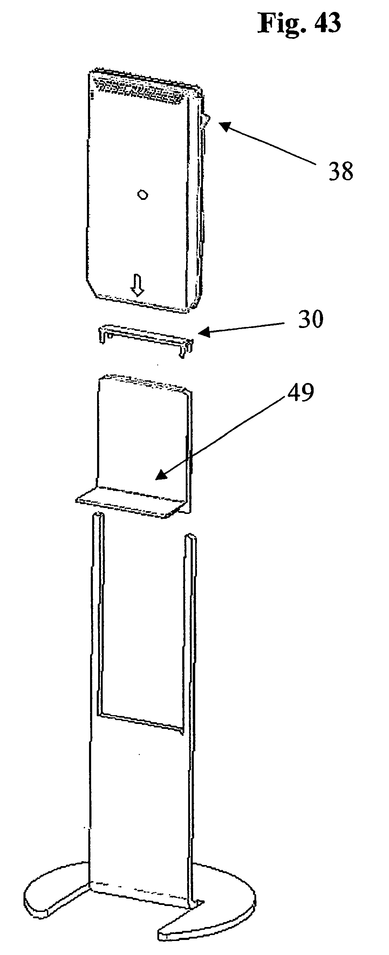

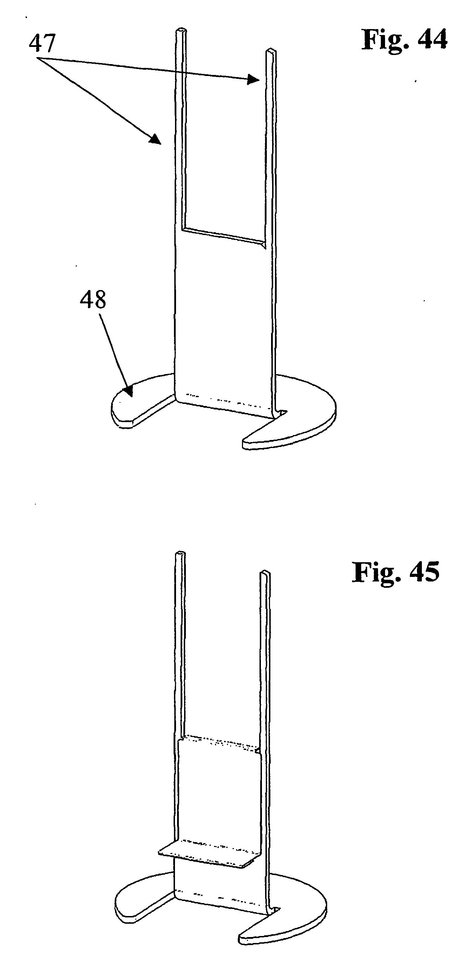

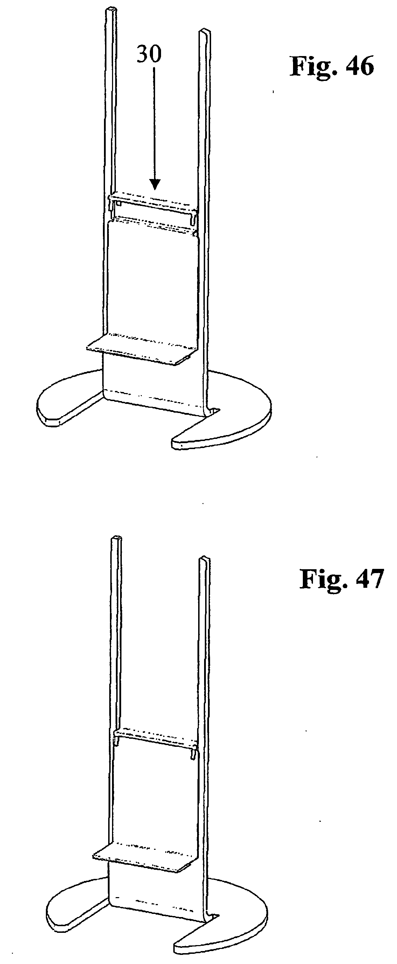

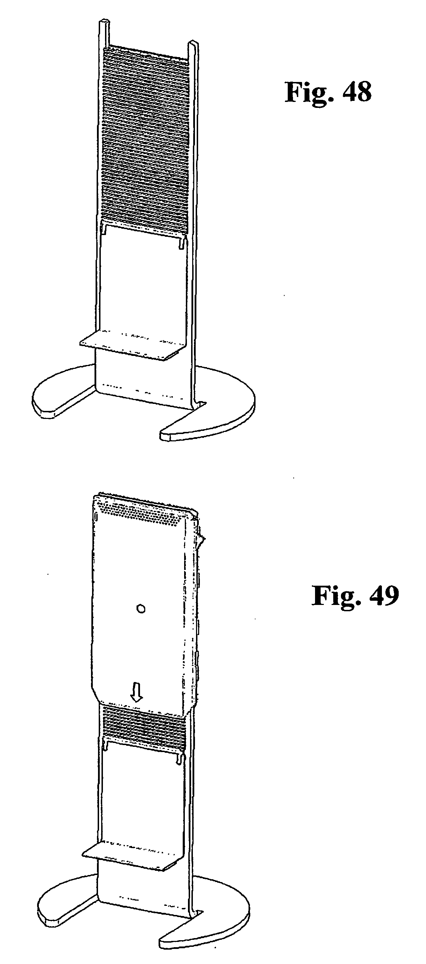

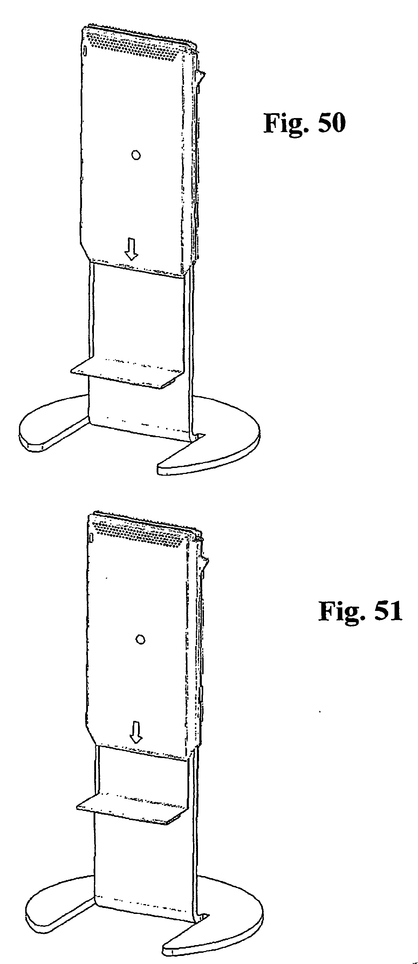

Fig. 43-51 illustrate the same mounting tool as shown in figure 39-42.

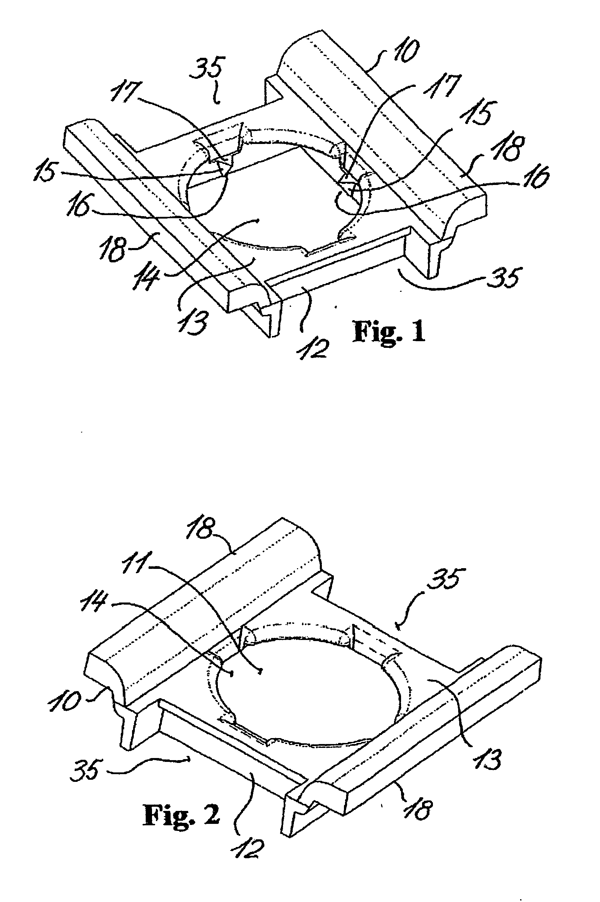

Figs. 1-3 illustrate a first embodiment of a testing device according to the invention comprising a holder 10 and a sheet- or plate-like test carrier or test member 11, which is a sheet or plate member containing for example colorimetric reagents. The holder 10, which may be made from plastic material by injection moulding, and the test sheet member 11 are produced separately and usually at different locations. Therefore, before the testing device may be used the test sheet member has to be mounted in the holder 10.

The holder 10 shown in Figs. 1-3 comprises a substantially rectangular frame 12 having inner side walls defining a passage having a cross-section corresponding to the size and shape of the test member 11. The upper end of the passage defined by the frame 12 is partly covered by an upper wall 13 defining an exposure opening 14 therein. The bottom surface of the upper wall 13 defines an abutment surface for the sheet-like test member 11 when mounted in the holder 10.

Oppositely arranged inner side wall parts have tooth-like projections 15 formed thereon. Each projection has a sloping leading edge 16 and a support surface or trailing surface 17, which is opposite to and substantially parallel with the bottom surface of the upper wall 13. Wing-like flanges 18 extending outwardly from opposite sides of the rectangular frame 12 are used for guiding the testing device along a path of movement or processing path in an automatic analyser, not shown.

A plate or sheet-like test member 11, such as a sheet of chemistry paper with a thickness of about 0.4 mm, may be mounted in the holder shown in Fig. 1 simply by pushing the test member upwardly trough the frame 12 towards the bottom side of the upper wall 13. The edge portions of the sheet-like test member 11 thereby come into engagement with the leading edges 16 of the projections, whereby these edge portions are locally compressed. Preferably, the thickness of the test member 11 corresponds to the axial distance between the abutment surface formed by the bottom surface of the upper wall 13 and the opposite supporting surfaces 17 of the projections 15. In such case the test member may be safely retained in position in the holder 10 between the bottom surface of the wall 13 and the opposing supporting surfaces 17 of the projections 15. When the testing device has been introduced into an analyser a liquid sample to be tested may be applied to the surface of the test member 11 exposed in the opening 14, and a colorimetric reaction may be read by the analyser and translated into a test result.

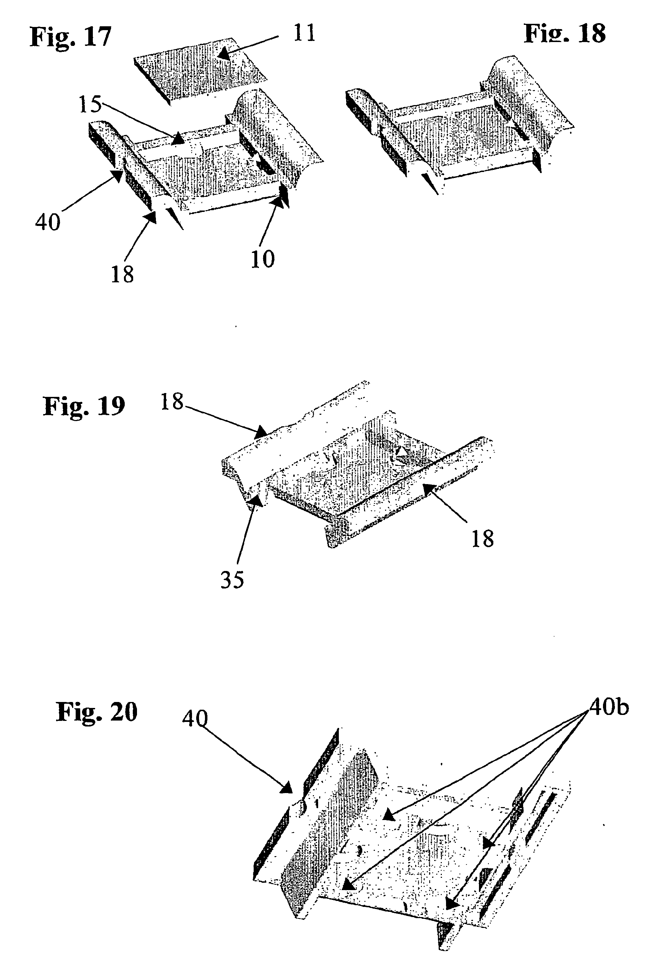

Figure 17 illustrates another embodiment of the testing device according to the invention. This embodiment comprises an upper side and a lower side in relation to an analysis instrument. The flanges 18 are positioned on the upper side of the holder and the retaining means are positioned and shaped so as to allow insertion of a plate or sheet-like test member 11, such as a sheet of chemistry paper, from the upper side of the holder (which is the opposite direction compared to the loading of a test member in the embodiment of figure 1). Figure 20 shows the same embodiment from the lower side.

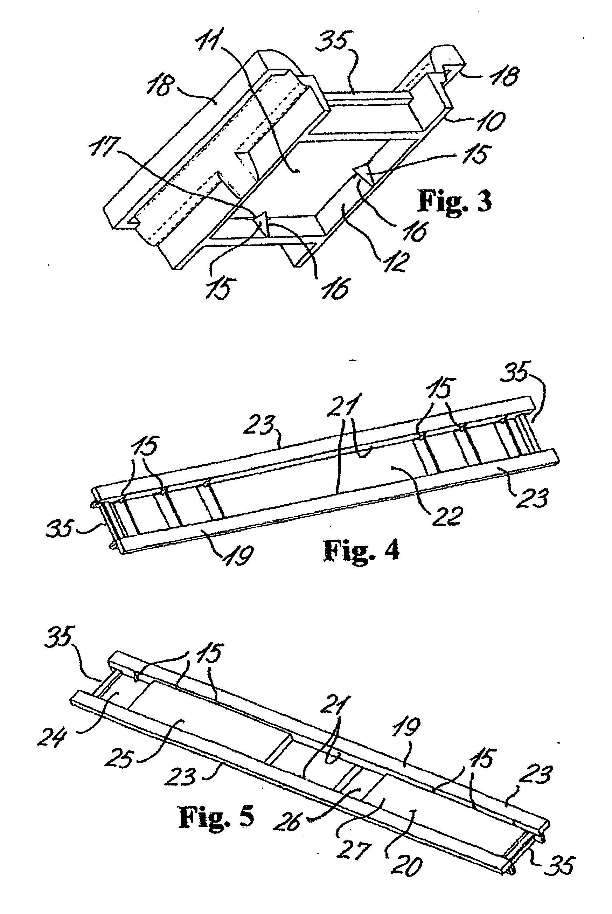

Fig. 5 illustrates another embodiment of the testing device according to the invention comprising a channel-shaped holder 19 shown in Fig. 4 and an elongated strip-type test member 20 having varying thicknesses along its length. The channel-shaped holder 19 has a pair of opposite side walls 21 and a connecting bottom wall 22 defining an inner abutment surface corresponding to the bottom surface of the upper wall 13 in the embodiment described above in connection with Figs. 1-3. A number of tooth-like projections 15 corresponding to those described above in connection with Figs. 1-3 are formed on the opposite inner surfaces of the side walls 21. A pair of oppositely directed flanges 23 extends from the upper edges of the side walls 21 and serves as guide flanges when the testing device is processed in an analyser.

As shown in Fig. 5, the lateral stick or test member 20 comprises various longitudinal sections including various layers and being of different thicknesses. These sections may, for example, include an application section 24 to which a liquid sample to be tested may be applied, a transfer section 25 for adding selected chemicals to the liquid sample and for transferring the same to a reading section 26 at which a colorimetric reaction may be read by an automatic analyser, and a collecting section 27 for receiving the liquid sample. Therefore, the various sections of the stick or test member 20 have different thicknesses. As apparent from Fig. 5 the thickness of the application section 24 substantially corresponds to the distance between the abutment surface defined by the bottom 22 and the supporting surface 17 of the adjacent projections 15, while the thicknesses of sections 25 and 27 substantially exceed that distance. This means that the projections 15 adjacent to the sections 25 and 27 bite into the edge portions of these sections and thereby retain them in the desired position in relation to the holder. Preferably, the distance between the abutment surface defined by the bottom 22 and the supporting surface of the projections 15 correspond to the thickness of the bottom layer of all of the sections 24-27.

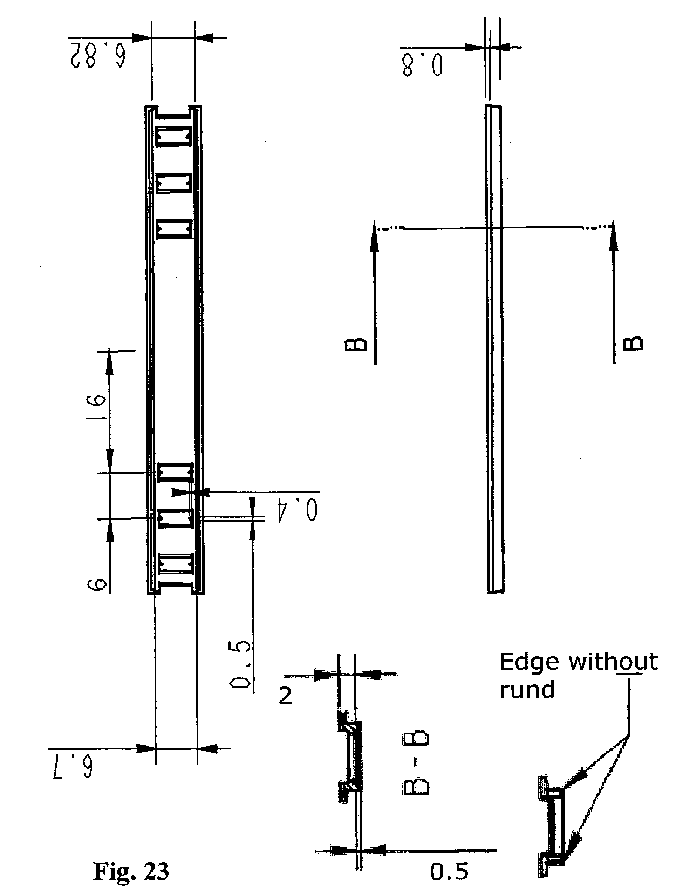

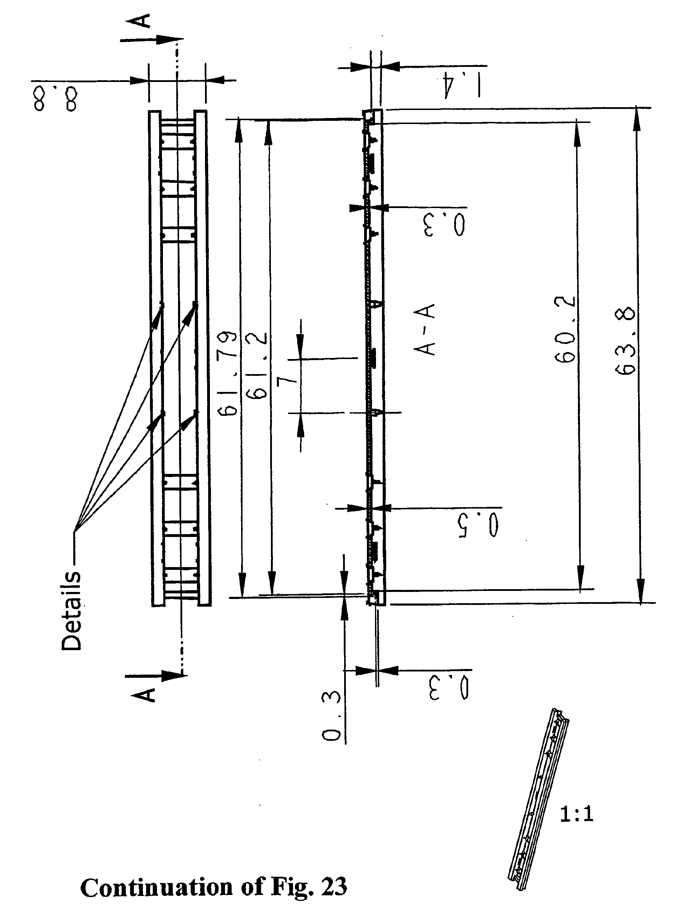

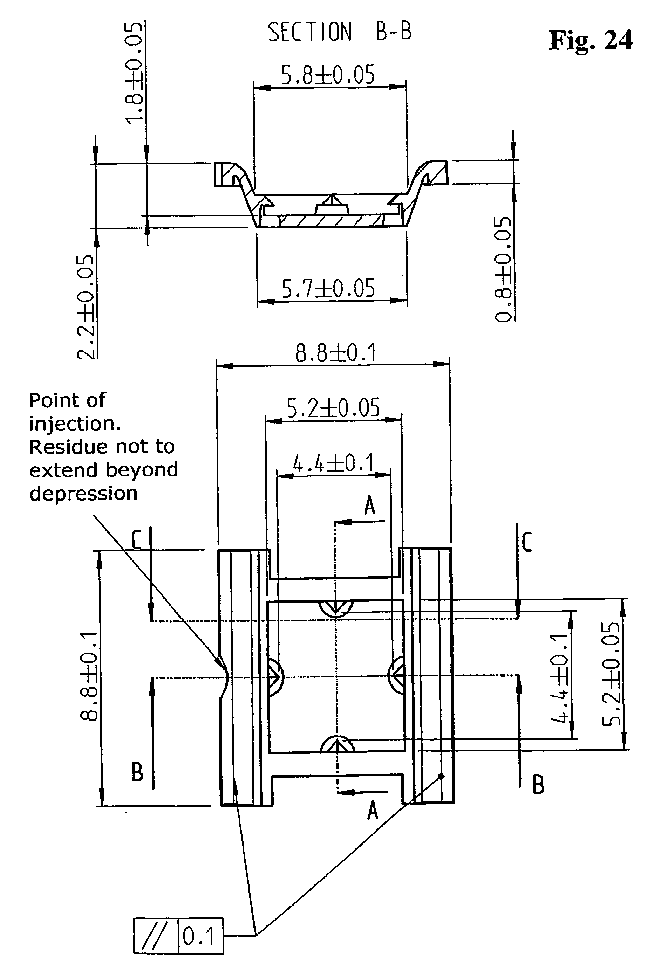

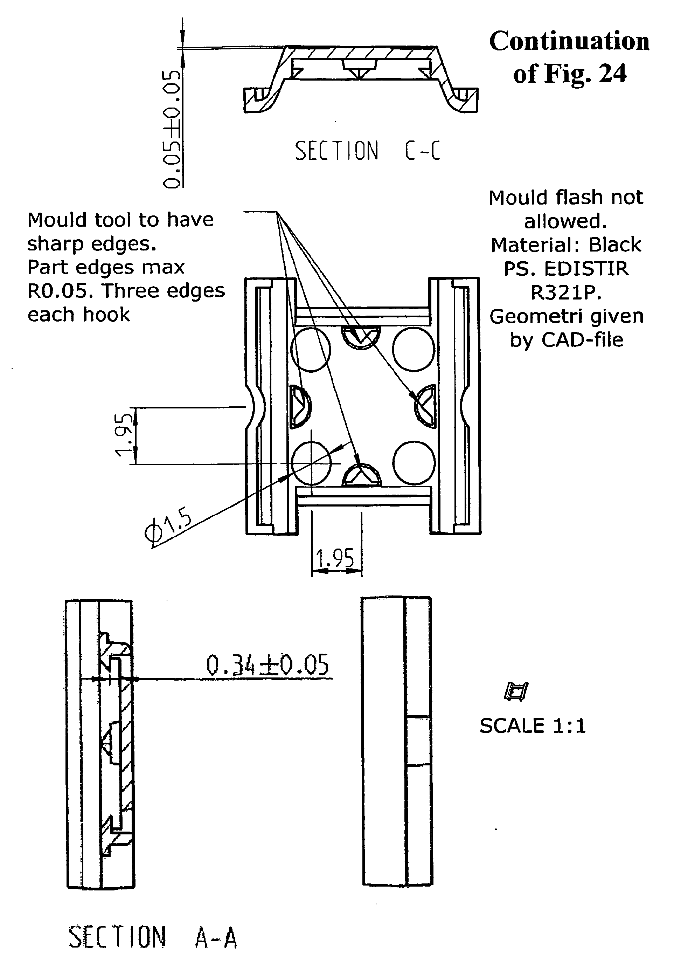

The stick frames shown in figure 23 and 24 provide and facilitate handleability to the chemistry pad.

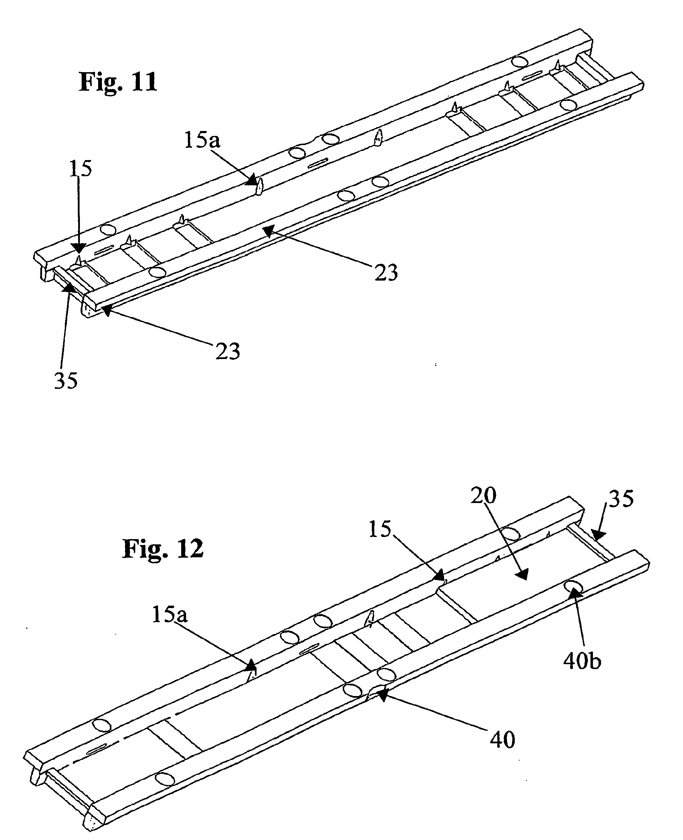

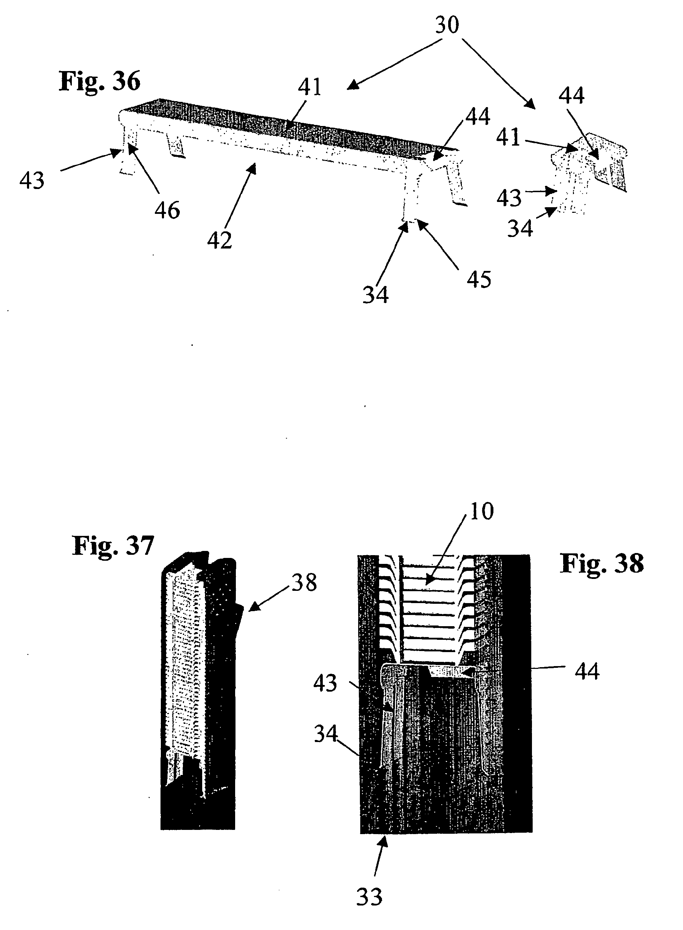

The life cycle of a frame from production to waste is described below: Firstly the frame is manufactured by injection moulding. There after the chemistry pad is pressed into the frame thus assembling the chemistry pad and frame, now forming a dry stick [DS], shown in figure 12 and figure 18. The dry sticks are stacked in appropriate numbers and inserted in to a cartridge. A formed sheet of preferably stainless steel is inserted under the stack forming a no-return floating bottom, securing the stack at any stack height, shown in figure 36. When the cartridge and the dry sticks have been assembled the cartridge is wrapped in protecting seam-welded bags, packaged in boxes and put on stock, while kept cold at 5°C. Thereafter the box is transported and distributed to the end-user, still kept cold. When the box arrives to the end-user, the end-user also has to make sure that the boxes containing the cartridges are stored in a cold storage such as a refrigerator. Single cartridges are retrieved from the cold storage and brought to the analysis instrument and inserted in the storage.

The chemistry has two formats, lateral (LS) flow and colorimetric (CS):
The lateral flow chemistry consists of a bottom foil with nitrocellulose and glue on which dosage, reaction and suction fiber pads are placed. A tape is placed on top, except at the dosage area. The chemistry is 5 by 60 mm and up to 1.6 mm high. The position of the reader-line is approximately in the middle.
Preferably somewhere between 25 mm and 39 mm such as 34 or 35 mm from the leading edge.
The colorimetric chemistry is formed by a 5 by 5 -mm fiber-pad. The pad is approximately 0.34-0.6 mm thick.

The material preferably used in the manufacturing of the frames is Polystyrene [PS]. It has been chosen as it has a low cost per volume and a high stiffness modulus. Furthermore it has a high surface tension towards milk, higher than Polyethylene [PE], reducing the risk of the milk seeking out in the gap between frame and chemistry.

The frame are preferably injection moulded. The geometry can be realized in injection tooling, without complexity e.g. separately moving cores etc. Due to the waste numbers needed, the production tooling will have several cavities - maybe as many as 64, and will utilize hot-runners and micro injection-nozzles. The tooling produces no runners and inlet-parts, meaning that there is need to separate and recycle scrap.
The point of injection is placed in an indention of the geometry to allow some degree of undefined geometry, see figure 20.
The ejection pins are slightly prolonged, 0.05 mm, so that wear and tolerances can be taken up without causing protrusions on the frame.
The stick assembling equipment checks each frame for faults, e.g. dimensions exceeding tolerances and incomplete geometry, and expels faulty frames. This could be done utilizing vision systems and/or laser grids.

The chemistry paper is mounted in the frame simply by a pressing motion with an appropriate shaped plunger. No-return hooks (15) placed on the walls of the frame secure the chemistry by positive engagement, shown in figure 1 and 4.
The bottom plastic foil of the lateral chemistry flips under the hooks, although it has cut/deformed to some degree. Frames were realized in soft tooling and assembly of chemistry carried out. It was found that the chemistry at the reader-line did not relate to the bottom of the frame as it bended, which affects the focus/precision of the reader. Therefore rib-protrusions 15a are added, which have a transversal distance smaller than the width of the chemistry, thus retaining the pad.
The colorimetric fiber pad is partly formed around and under the hooks, thus retained inside the holder.

The first embodiment of the colorimetric frame, had the chemistry inserted from underneath see figure 1 and 2. This design presents the top of the chemistry with less tolerance of the level, and has a higher flexibility regarding different/changing pad thickness and less critical retaining function, as the hooks have a better leading angle. The second embodiment of the colorimetric frame, shown in figure 17 and 18 may be preferred if the concept of dosage is positive contact of the dosage device to the chemistry instead of a noncontact of the dosage device. A positive contact concept of the dosage device calls for support from underneath of the pad.
Thus, the advantages of having a frame loaded from beneath, shown in figure 1 and 2, is that the distance to a reader (not shown) is not dependent on the thickness of the chemistry.
The advantages of having a frame loaded from above, shown in figure 17 and 18 is that the method of applying fluid on to the chemistry can be done by direct contact. Because the chemistry is resting against the bottom of the frame it is not removed when the application tool apply a force on to the chemistry.

The design of the frames aim to have:
- Lowest possible cost
- Ease of automated production
- High reliability of AI - avoid malfunctions and influence of the precision of measurements
- Small physical dimensions
- Ease of disposal
- Lowest possible environmental impact
- Ease of development, same design paradigm for both frames
- Same level of dosage for both DS
- Same level of reading for both DS

The overall height, is preferably 2.5 mm, of the LS frame, and may be determined by the chemistry used in the LS - the chemistry is preferably 1.6 mm thick. The floor of the LS-frame is preferably 0.6 mm thick, leaving a clearance of preferably 0.3 mm from the top of the frame to the chemistry. The stack-height of the LS is the full 2.5 mm.
The stack-height of the CS may be reduced to 1.4 mm, by preferably reducing the thickness of the frame body, utilizing the thinner chemistry. The overall height of the CS frame is preferably 2.2 mm. The height of the CS-frame is based on the distance from the upper side of the LS frame to the LS-read plan. Furthermore is the height of the CS-frame also depending on the thickness of a CS-stick and the thickness of the floor of the CS-frame.

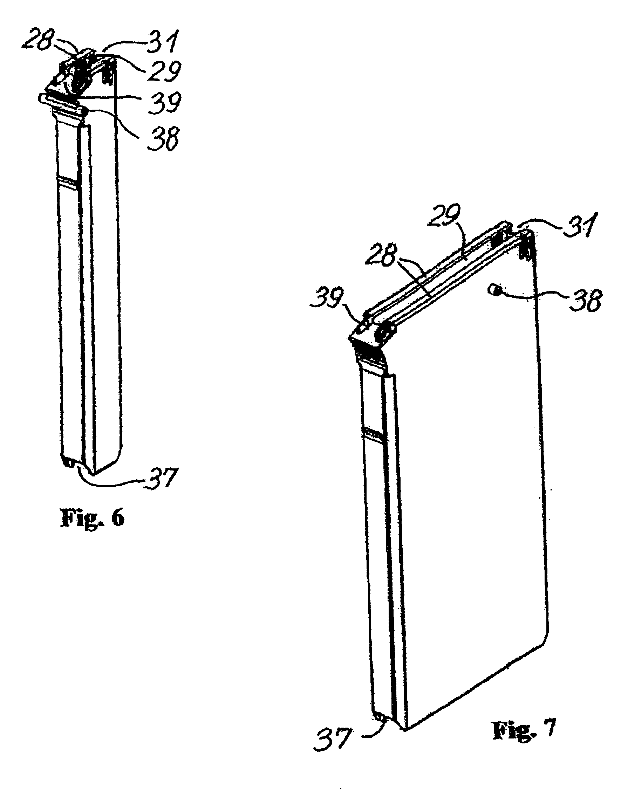

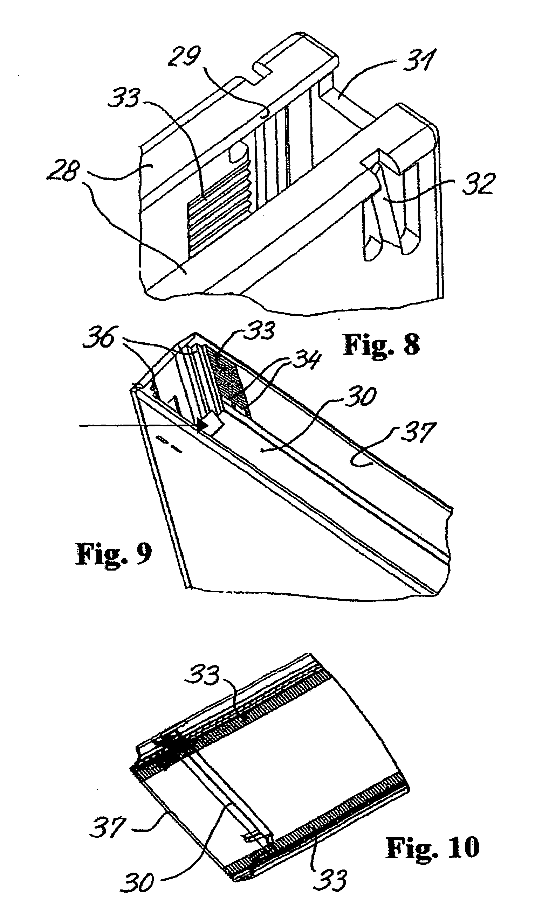

Figs. 6 and 7 illustrate storage containers or cassettes for containing a stack of testing devices of the type shown in Figs. 1-3 and 4-5, respectively. These cassettes are adapted to be mounted in an automatic analyser (not shown) so that the testing devices contained therein may be selective discharged for being processed in the analyser. Each of the cassettes shown in Figs. 6 and 7 has a tubular shape with an inner rectangular cross-section substantially corresponding to the outline of the testing devices to be housed therein (Figs. 2 and 5, respectively), and an open bottom or bottom opening 37 as shown in Fig. 9. The upper end of each of the storage containers or cassettes shown in Figs. 6 and 7 is partly covered by a pair of opposite, substantially parallel flanges or ledges defining a transversely extending space 29 there between (Fig. 8).

It is important that colorimetric as well as lateral sticks are guided securely and that they are as easy to handle as possible all the way from production until use in the analysis instrument.
The vertical guidance has to be so robust that the sticks are not erroneously oriented, before they are taken out by the stick mover horizontally. It is necessary for the cartridge to be designed in a way that enables the stick mover to run into an integration surface and be presented to sticks in the same way each time.

During production, transportation and handling of the cartridge with sticks, the cartridge has to be able to withstand all possible ways of treatment, which may include pushes, strokes and even drops, but which must not make the sticks to be erroneously oriented. The LC (lateral cartridge) preferably contains 50 sticks, and the CC (colorimetric cartridge) preferably 100 sticks.

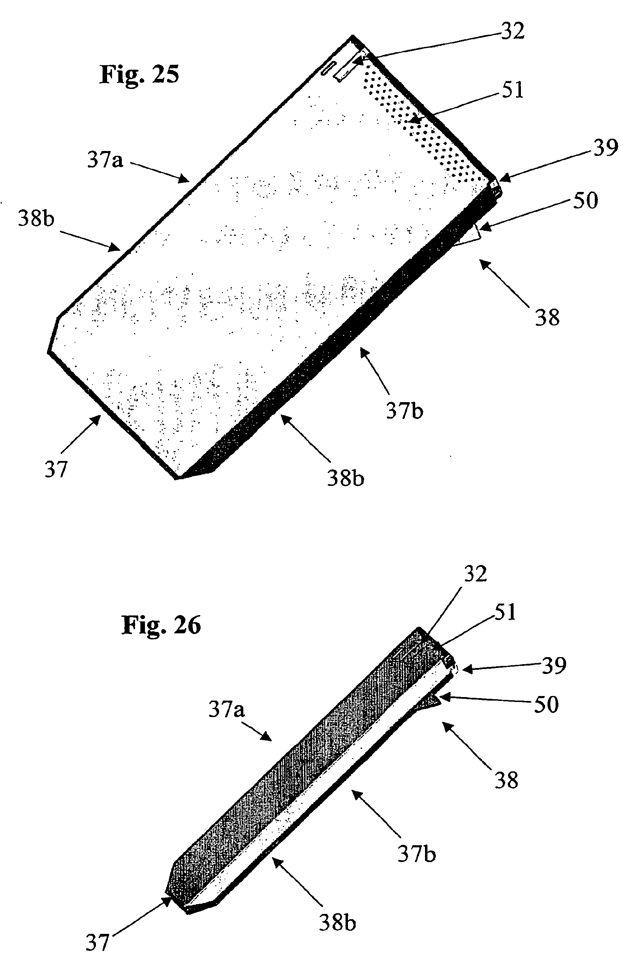

Due to various physical designs of colorimetric and lateral sticks, two types of cartridges are available. The two types are called Colorimetric Cartridge [CC] and Lateral Cartridge [LC], respectively. Apart from the depth, the two cartridges are almost identical. The two cartridges can be seen in figure 25 and figure 26.

Preferably a cartridge consists of two injection-moulded shells, which preferably have been ultrasonic welded together. The shells are preferably made of impact modified Polystyren, which has been chosen due to the favourable price and the mechanical qualities desired, both regarding strength/stiffness and welding.

In the following, the cartridge and the parts, which are integrated in the cartridge, are described more closely and will apply for both the CC and the LC.

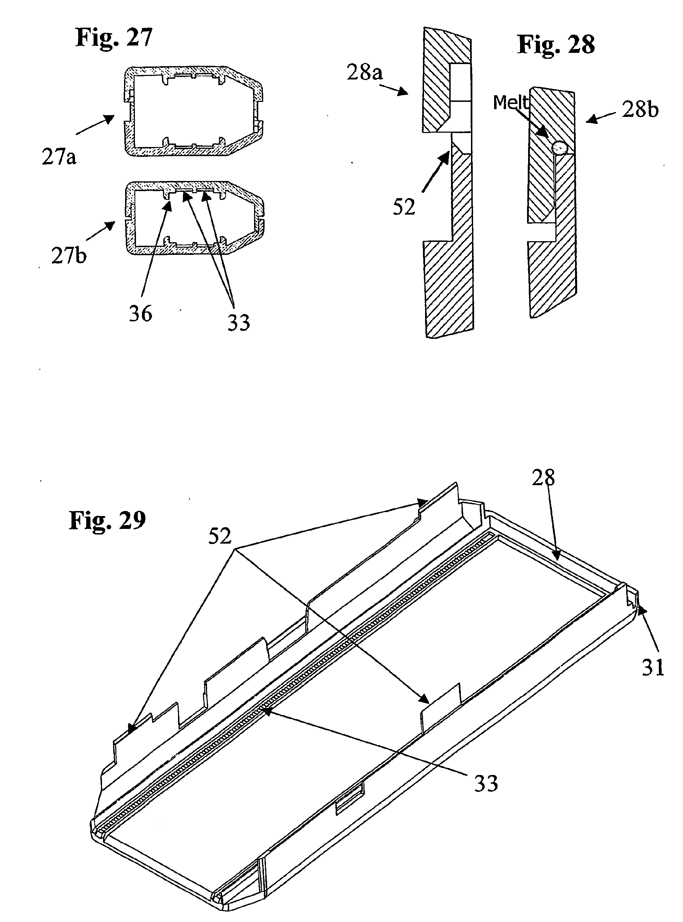

A cartridge preferably consists of two injection-moulded shells/sides, which preferably have been ultrasonic welded together, see figure 27 and 28.
Each shell has three energy directors, see figure 29 (six per cartridge), which have been placed male/female alternately.
The welding takes place by way of a specially manufactured welding horn and a fixture on a 20 kHz welding machine.
The welding time including fixing time is preferably approx. 1.5 second.
In the production the welding may take place fully automated inline with an injection-moulding machine.

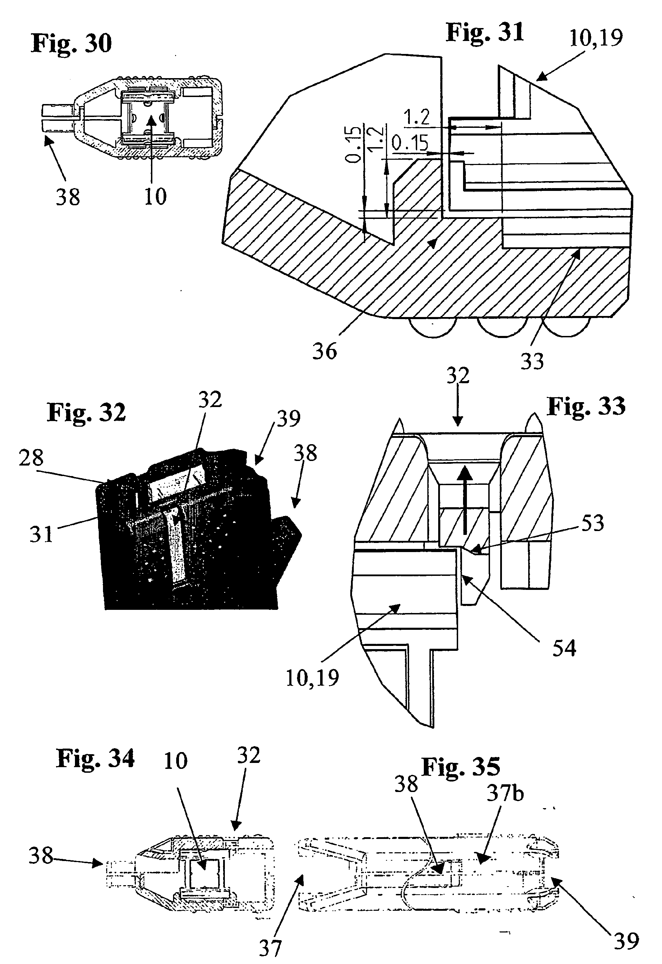

Each cartridge comprises a vertical guide-way for guiding sticks within the cartridge. Preferably the nominal air around the stick is 0.15 mm all the way round (thus 0.3 mm in each direction).The width of the guide in the edge is preferably 1.2 mm. See figure 31.
To make sure that the sticks can be handled smoothly without being squeezed by the cartridge and without capsizing (lateral sticks may have a tendency of that), the welding has to be as precise as possible.

Furthermore, each cartridge preferably comprises a spring lock in order to ensure that the sticks cannot be removed from the cartridge in case of shocks when handling, in such cases the sticks are held back by a spring lock built into the cartridge. Shown in figure 32 and 33.

The blocking of the spring locks can preferably only be removed, when the stick is taken out of the stick mover.

Below follows some data and specifications of the cartridges. The cartridges are preferably welded in impact modified Polystyren. Some of the advantages of using Polystyren as material are because the Polystyren has good mechanical properties and is suitable for ultrasonic welding and it is also an Inexpensive material.
The preferred physical data for a Lateral Cartridge are:
- Volume: 2 x 21500 mm³
- Weight: 2 x 22.6 g
- Main dimensions (L x W x D): 160 x 13.2 x 25 mm

And the preferred physical data for a Colorimetric Cartridge are:
- Volume: 2 x 8200 mm³
- Weight: 2 x 8.6 g- Main dimensions (L x W x D): 160 x 13.2 x 25 mm

To ensure that the sticks in the cartridge are always in the top of the cartridge, and that the stack of sticks is kept in place, the movable bottoms shown in figure 36 have been used. As shown in Figs. 9 and 10, each of the storage containers or cassettes contains a movable bottom plate or support plate 30 and when loaded with a stack of testing devices (Figs. 2 and 5) such stack is arranged between the bottom plate 30 and the inner surfaces of the flanges 28, so that the uppermost testing device in the stack is in abutting engagement with the flanges 28 and aligned with a discharge opening 31 formed in the adjacent cassette wall. A resilient finger 32 formed integrally with the cassette wall is frictionally engaging with the uppermost testing device in the stack so as to avoid unintentional discharge of the same trough the discharge opening 31. Rows of teeth or ratchet teeth 33 formed on opposite inner walls of the storage container or cassette are engaging with pawl members 34 (Fig. 9) connected to the movable bottom plate 30 so as to allow movement of the bottom plate 30 in one direction, only, namely towards the flanges or ledges 28.

The movable bottom 30 may be made of bent sheet metal, so that its shoulders are flexible and act as a lock. The lock runs against four internal one-way stairs in the cartridge (see illustration in figure 37 and 38).

The movable bottom may be made of stainless steel preferably by way of laser cutting and bending.

When the cartridge has been emptied for sticks, and the bottom is in the top of the cartridge, a bend preferably approximately 45-degrees 44, ensures that a stick mover pawl will slide over the bottom. The bottom is guided between the four legs 43 and the side-guidance of the stairs shown in figure 38.

Some data and specifications for the movable bottom are described below. The movable bottoms are preferably made out of 0.10 mm stainless spring steel, AISI 301. The bottoms are first cut by laser cutting/photo etching items for function models. There after they are bent into the final shape by specially manufactured tools.
Thus, the preferred method for making a movable bottom device comprises the steps of;
- laser cutting(small batches) or punching (large batches), and
- bending sheet metal

The movable bottom device shown in figure 36 comprises a testing device support side 41, a plunger abutment side 42, and at least two legs 43.

The movable bottom device further comprises a slope 44 characterised in that a part of the support surface slopes downwards in relation to the stick support side, for guiding a testing device remover pawl.
The legs of the movable bottom device comprise at least one curvature 45 creating a base area 46 and a pawl 34.

The base area 46 is the part of the leg being close to the support surface, whereas the pawl area 34 is the part of the leg that is on the opposite side of the at least one curvature in relation to the base area. The pawl area being in a different angle in relation to the rest of the leg.

Preferably, at least two pawl members 34 differing in length are associated with each row of teeth 33, and said difference in length is smaller than the pitch of the row of teeth 33, preferably half the pitch or the pitch divided by the number of pawl members for each row of teeth, if more pawl members are used for each row of teeth. This means that the movable bottom plate 30 may be retained in positions having a mutual spacing being smaller than - such as half - the pitch of the row of teeth 33. Thus, such small spacing may be obtained without requiring close tolerances in moulding the row of teeth, while the lengths of pawl members, made for example from metal, may rather exactly be cut to the desired lengths.

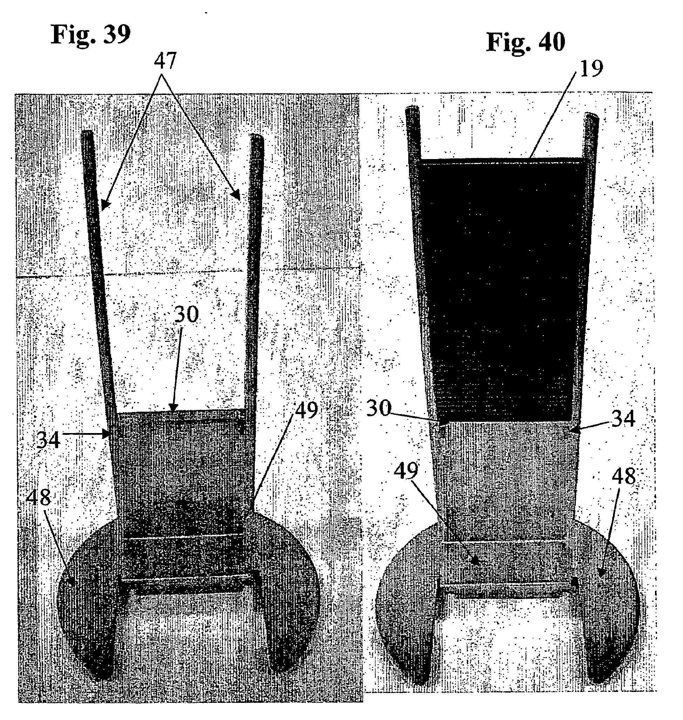

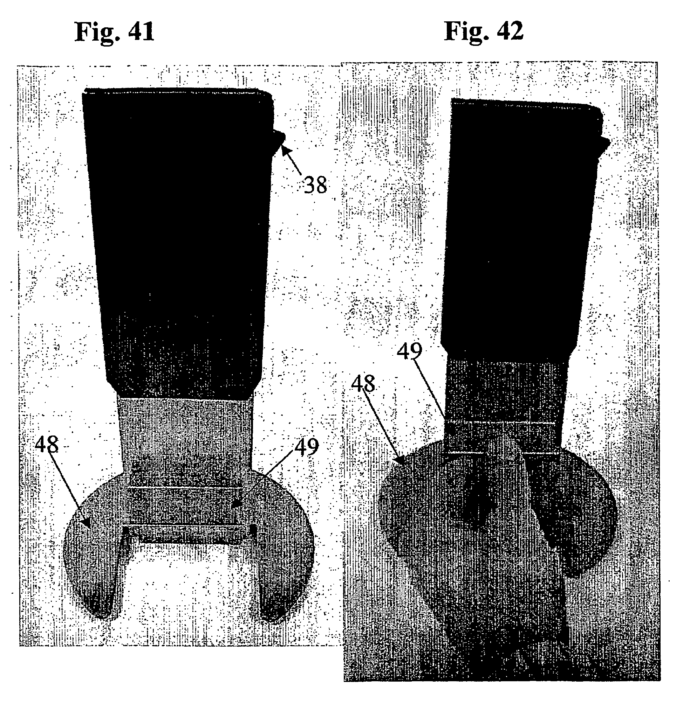

In order to facilitate the transportation of sticks and for making it easy for a user to handle the sticks, the sticks are mounted in a cartridge. The section below describes the method of how to load a cartridge with preferably 50 lateral or 100 colorimetric sticks by using a loading device, see figure 39-42 for illustration:

The testing devices are stacked and the stack is introduced in the storage container or cassette in the following manner:
A mounting tool (not shown) comprises a base and a pair of upwardly extending guide legs. These legs are dimensioned such as to be received in opposite recesses or cut-outs 35 formed in the testing devices (Figs. 1-3 and Figs. 4 and 5, respectively). As a first step, the movable bottom plate 30 is positioned on the base of the tool, where after the desired number of testing devices is stacked on top of the bottom plate such that the guide legs of the mounting tool are received in the opposite recesses 35 of the testing tools.
Finally, the tool with the stacked testing devices is inserted into a cassette via the open bottom or bottom opening 37 and pushed towards the flanges or ledges 28. When the pawl members 34 of the bottom plate 30 have come into engagement with the rows of teeth 33, the tool may be withdrawn from the cassette, while the movable bottom plate 30 and the stacked testing devices remain therein, because the pawl members 34 of the bottom plate 30 engage with the corresponding rows of teeth 33 on the inner surfaces of the cassette.
As shown in Fig. 9 a longitudinally extending corner guide 36 is formed in each inner corner of the cassette for guiding the complementary shaped corners of the flanges 18 and 23, respectively, of the stacked testing devices when being displaced upwardly through the tubular cassette.

A method useable for loading a cartridge is described below.
Firstly a movable bottom is placed in a temporary fixture between the two guide legs 47. Secondly, a number of lateral or colorimetric sticks are placed in the fixture on top of the movable bottom and also between the two guide legs. The recesses in the end of the stick guide the sticks along the columns, see figures 23 and 24. When the sticks are in place a cartridge is taken down to the fixture and guided so that the two guide legs penetrate the cartridge from the bottom. Preferably the cartridge is pushed downwards until the one-way stairs inside the cartridge gets in contact with the pawls of the movable bottom. In order to get the sticks to the top of the cartridge, the position of the cartridge is secured, while the auxiliary plate of the fixture is being pushed upwards. To secure that the stack of sticks are kept in place, cartridges may preferably be loaded and unloaded in a loading device.

The lifting device (auxiliary plate) in the loading device is preferably at least partly positioned between the two guide legs , and is preferably of the same size or smaller than a testing device. The distance between the first and the second guide leg is adjusted so that a testing device can be guided between the first and second guide legs. The mounting tool and the method are preferably designed so that the loading can be automatically performed.

A cassette of the type shown in Figs 6 and 7 loaded with stacked testing devices may be mounted in an automatic analyser such that a spring biased piston or plunger is moved through the bottom opening 37 of the cassette into engagement with the movable bottom plate 30 therein for biasing the stack of testing devices towards the bottom side of the flanges or ledges 28. The cassette may be retained in position by means of oppositely extending mounting pins 38. The upper testing device is retained in position in alignment with the discharge opening 31 by the resilient finger 32 to avoid unintentional discharge. However, the analyser may move the upper testing device into the processing path of the analyser by means of a reciprocating carrier pin of the analyser movable via a funnel-shaped entrance 39 (Figs. 6 and 7) into the transverse space 29 and into engagement with the upper testing device so as to transfer the same to the processing path of the analyser via the discharge opening 31. When the upper testing device has been discharged from the cassette the stack of testing devices within the cassette will be moved a step upwardly under the influence of the biasing piston or plunger, where after the discharge operation may be repeated. When a testing device has been used for analysing a liquid sample, the device may be discarded or the test member 11, 20 may be removed from the holder 10, 19, where after the holder may be reused together with a fresh test member.

The cassettes shown in Figs. 6 and 7 may be made from two laterally reversed halves which may be welded or glued together or irreversibly interlocked by mechanical snap locking means. These halves may possibly be integrally moulded such that they are hinge connected along one side.

The cassettes or cartridges shown in figure 6,7,25 and 26 may further comprise a front edge surface 37a and a back edge surface 37b comprising at least one energy director, shown in figure 29. The energy director(s) of the left half front edge surface and back end surface is located in relation to guide ways on the right half front edge surface and back edge surface for receiving the energy director(s). These energy directors facilitate the joint of the two halves during ultrasonic welding.

It should be understood that the projections 15 formed on the holder 10, 19 may be of any shape as long as they are able to allow insertion of the test member 11, 20 into the holder and to retain the test member therein in the desired mutual position. As an example, the projections 15 may be in the form of pins extending obliquely towards the abutment surfaces defined by the walls 13, 22. Furthermore, the projections may be positioned differently spaced from these abutment surfaces so that test members 11, 20 of different thicknesses may be safely retained in one and the same holder 10, 19.

Fig. 12 illustrates another embodiment of the testing device according to the invention comprising a channel-shaped holder 19 shown in Fig. 5 and 11 and an elongated strip-type test member 20 having varying thickness along its length. The channel-shaped holder 19 has a pair of opposite side walls 21 and a connecting bottom wall 22 defining an inner abutment surface corresponding to the bottom surface of the upper wall 13 in the embodiment described above in connection with Figs. 1-3. A number of tooth-like projections 15 corresponding to those described above in connection with Figs. 1-3 are formed on the opposite inner surfaces of the side walls 21. A pair of oppositely directed flanges 23 extends from the upper edges of the side walls 21 and serves as guide flanges when the testing device is processed in an analyser.
A number of rib-shaped projections 15a are formed on the opposite inner surfaces of the side walls 21, in order to obtain a better fit of the chemistry in the frame.

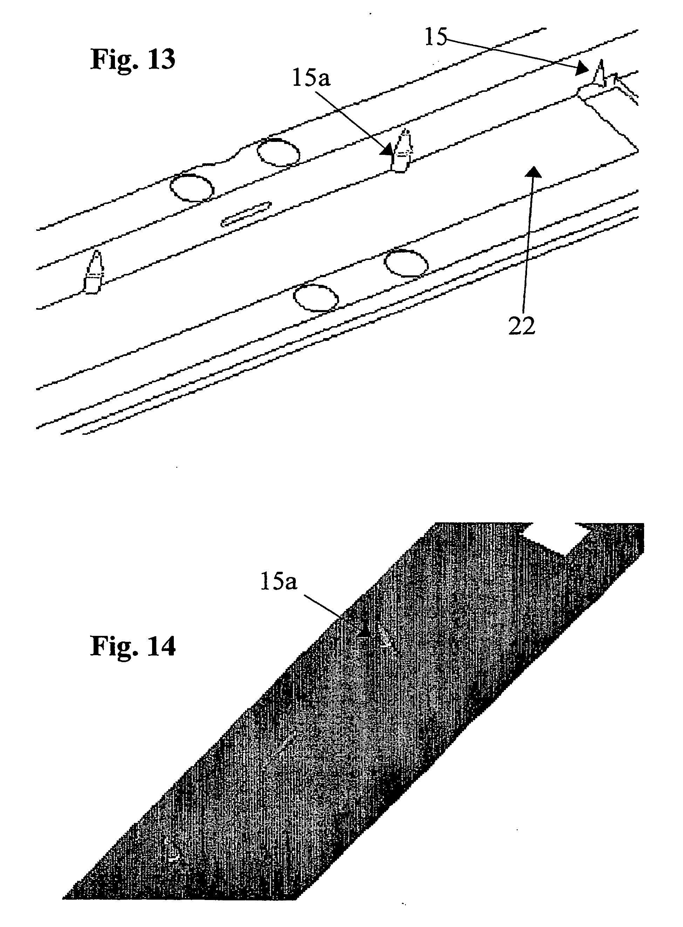

Fig. 13 and 14 are a magnification of the holder in figure 11 and 12. These figures illustrate in relation to the bottom wall 22, two vertical ribs 15a formed on the opposite inner surface of the side walls.

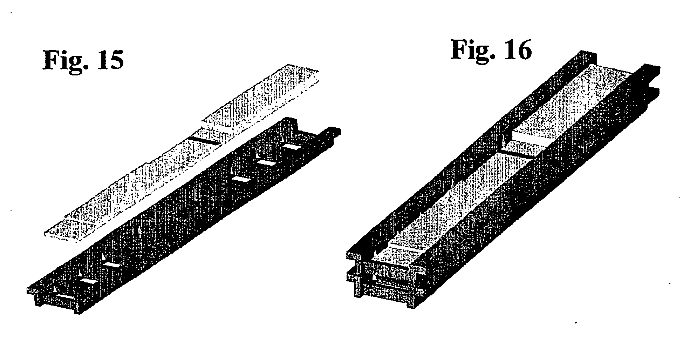

Fig. 15 illustrates the holder and test stick shown in figure 11 and 12 but here the stick is separated from the holder.

Fig. 16 illustrates two holders shown in figure 11 and 12, piled on top of each other.

Figs. 17-20 illustrate another embodiment of a testing device according to the invention comprising a holder 10 and a sheet- or plate-like test carrier or test member 11, which is a sheet or plate member containing for example colorimetric reagents. The holder 10, which may be made from plastic material by injection moulding, and the test sheet member 11 are produced separately and usually at different locations. Therefore, before the testing device may be used the test sheet member has to be mounted in the holder 10.

The holder 10 shown in figs. 17-20, comprises a substantially rectangular frame 12 having inner side walls defining a pit having a cross-section corresponding to the size and shape of the test member 11. The bottom of the pit is defined by the frame 12 and is partly covered by a lower wall 22 defining an abutment surface. The top surface of the lower wall 22 defines an abutment surface for the sheet-like test member 11 when mounted in the holder 10.

Oppositely arranged inner side wall parts have tooth-like projections 15 formed thereon. Each projection has a sloping leading edge 16 and a support surface or trailing surface 17, which is opposite to and substantially parallel with the top surface of the lower wall 22. Wing-like flanges 18 extending outwardly from opposite sides of the rectangular frame 12 are used for guiding the testing device along a path of movement or processing path in an automatic analyser, not shown.

A plate or sheet-like test member 11, such as a sheet of chemistry paper with a thickness of about 0.4 mm, may be mounted in the holder shown in Fig. 17-20, simply by pushing the test member downwardly trough the frame 12 towards the top surface of the lower wall 22. The edge portions of the sheet-like test member 11 thereby come into engagement with the leading edges 16 of the projections, whereby these edge portions are locally compressed.

Preferably, the thickness of the test member 11 corresponds to the axial distance between the abutment surface formed by the top surface of the lower wall 22 and the opposite supporting surfaces 17 of the projections 15. In such case the test member may be safely retained in position in the holder 10 between the top surface of the wall 22 and the opposing supporting surfaces 17 of the projections 15. When the testing device has been introduced into an analyser a liquid sample to be tested may be applied to the surface of the test member 11, and a colorimetric reaction may be read by the analyser and translated into a test result.

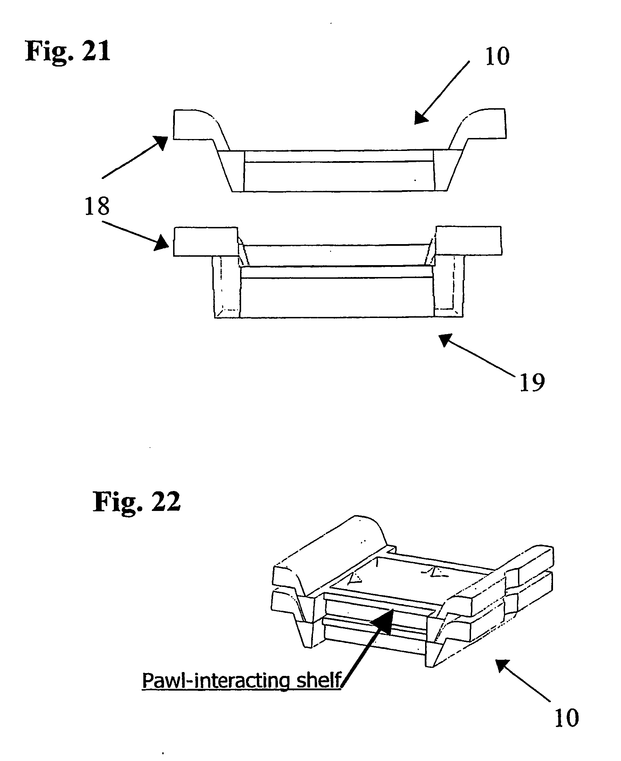

Fig. 21 shows the two different types of frames 10 and 19, in profile. The frames have wing-like flanges 18 extending outwardly from opposite sides of the rectangular frame 12 used for guiding the testing device along a path of movement or processing path in an automatic analyser, not shown.

Fig. 22 illustrates two holders shown in figures 1-3 or 17-20, piled on top of each other.

Fig. 23 shows the drawings of the third embodiments.

Fig. 24 shows the drawings of the fourth embodiment.

Fig. 25 shows a side view of a cartridge for the holder 19. The cartridge has a resilient finger 32, for preventing the stick holder to fall out during transportation of the cartridge. Also the cartridge has grip protrusions 51 on the upper half of the sides for increasing the friction between the hand and the cartridge when loading and unloading the cartridge. Furthermore the cartridge also has a external protrusion 38 located on the back surface in the vicinity of the upper discharge opening, on the same edge as the funnel-shaped entrance 39. The protrusion has an abutment surface 50.

Fig. 26 shows a side view of a cartridge for the holder 10. The cartridge has a resilient finger 32, for preventing the stick holder to fall out during transportation of the cartridge. The cartridge has grip protrusions on the upper half of the sides for increasing the friction between the hand and the cartridge when loading and unloading the cartridge. The cartridge also has a protrusion 38 on the same edge as the funnel-shaped entrance 39. The protrusion has an abutment surface 50, for providing a preferred vertical storage positioning during storage of the cartridge in the analysis instrument (not shown). The cartridge further comprises a hole, 38b in the wall 37b as well as in the wall 37a. These holes are preferably adopted for receiving retaining means (Not shown) for holding the cartridge in a loading position when the cartridge is being loaded in to an analysis instrument (Not shown). Thus facilitating the loading for a user.

Fig. 27 shows two cross-sections of the cartridge for the holder 10, the above cross-section shows the cartridge before assembly 27a and the lower cross-section show the cartridge after it is assembled 27b. The corner guide 36 and the rows of teeth or ratchet teeth 33 can also be seen.

Fig. 28 shows a magnification of the energy directors whereby the two cartridge halves are welded together by preferably ultrasonic welding.

Fig. 29 shows one of the halves of a cartridge for the holder 19, with three energy directors 52.

Fig. 30 shows a cross-section of a cartridge wherein a holder 10 is placed. Furthermore the abutment surface 50 of the protrusion 38 can be seen.

Fig. 31 shows a magnification of the grip protrusions 51, corner guide 36 and of the rows of teeth or ratchet teeth 33.

Fig. 32 shows a close up of the upper part of a cartridge illustrating the preferred placement of a resilient finger 32.

Fig. 33 and 34 shows a cross-section of the resilient finger, wherein the sloping abutment surface 53 for preventing holders to fall out during transportation of the cartridge but at the same time has the feature to release a holder when the holder is pushed against the resilient finger. The resilient finger further comprises a second level abutment surface 54, abutting the holders below the upper holder.

Fig. 35 shows a cartridge with the funnel-shaped entrance 39.

Fig. 36 shows two embodiments of the movable bottom plate having a support surface comprising a stick support side 41 and a plunger abutment side 42. The bottom plate further comprising four legs 43 having a base area 46 and a pawl area 34. Furthermore the bottom plate comprises a slope 44 in one of the end for guiding a stick remover pawl.

Fig. 37 and 38 show a cartridge for holders (10), wherein the placement of sticks and the movable bottom plate can be seen.

Fig. 39-42 show a mounting tool for loading cartridges with holders. The mounting tool comprises a pair of upwardly extending guide legs 47 and a base 48. The mounting tool further comprises a lifting device 49 for pushing the stack of holders towards the flanges 28 and for forcing the floating bottom plate into the cartridge so that the pawl area 34 of the legs of the bottom plate gets in contact with the rows of teeth or ratchet teeth 33 on the inside of the cartridge.

## Claims

1. A testing device for testing or analysing fluids and comprising
at least one sheet- or plate-like test member (11, 20) including analysis reagent and having opposite side surfaces surrounded by edge portions, and
a separately produced holder (10, 19) having retaining means for receiving and retaining the test member in a predetermined relative position in the holder,
wherein
said retaining means comprises an abutment surface (13, 22) engaging with one of said side surfaces of the test member and projections (15, 15a), which are positioned and shaped so as to allow insertion of the test member into the holder by moving the test member into engagement with said abutment surface while engaging with opposite edge portions of the test member,
and wherein
said holder has upper and lower complementary surfaces so as to allow stacking of a plurality of testing devices on top of each other, **characterised in that** said complementary surfaces are shaped so as to guide mutual displacement of stacked testing devices along a path of movement along one axis transversely to a longitudinal axis of the stack.

2. A testing device according to claim 1, wherein the projections (15) are tooth-shaped with pointed ends.

3. A testing device according to claims 1 or 2, wherein each of at least some of the projections(15) has a leading edge (16) forming a ramp sloping towards a plane defined by the abutment surface so as to facilitate insertion of the test member into the holder.

4. A testing device according to any of the preceeding claims, wherein each of at least some of the projections (15) has a trailing edge or surface (17) extending substantially parallel with and spaced from a plane defined by the abutment surface.

5. A testing device according to any of the preceeding claims, wherein the projections are positioned so as to be differently spaced from the plane defined by the abutment surface.

6. A testing device according to any of the preceeding claims, wherein the holder (19) is a channel-shaped member having an inner bottom surface (22) defining said abutment surface and opposite inner side surfaces (21) from which projections (15) extend in opposite directions.

7. A testing device according to claim 6, wherein the test member (20) is an elongated member of the "lateral flow stick" type, in which the fluid to be tested is supplied at one end (24) of the elongated test member.

8. A testing device according to any of the claims 1-5, wherein the holder (10) is frame-shaped and defines an opening (14) therein, the abutment surface (13) extending around and adjacent to said opening.

9. A testing device according to any of the claims 1-8 for use in colorimetric testing of milk.

10. A holder for use in a testing device according to any of the claims 1-9, said holder (10, 19) comprising means for receiving and retaining a sheet- or plate-like test member (11, 20), which has opposite side surfaces surrounded by edge portions, in a predetermined relative position in the holder, said retaining means comprising an abutment surface (13,22) for engaging with one of said side surfaces of the test member and projections (15, 15a), which are positioned and shaped so as to allow insertion of the test member into the holder by moving the test member into engagement with said abutment surface while engaging with opposite edge portions of the holder,
wherein the holder has upper and lower complementary surfaces so as to allow stacking of a plurality of holders on top of each other, **characterised in that** said complementary surfaces are shaped so as to guide mutual displacement of stacked holders along a path of movement along one axis transversely to the longitudinal axis of the stack.

11. A holder according to claim 10, wherein the projections (15) are tooth-shaped with pointed ends.

12. A holder according to claim 10 or 11, wherein each of at least some of the projections has a leading edge (16) forming a ramp sloping towards a plane defined by the abutment surface so as to facilitate insertion of the test member into the holder.

13. A holder according to any of the claims 10-12, wherein each of at least some of the projections has a trailing edge or surface (17) extending substantially parallel with and spaced from a plane defined by the abutment surface.

14. A holder according to any of the claims 10-13, wherein the projections are positioned so as to be differently spaced from the plane defined by the abutment surface.

15. A holder according to any of the claims 10-14, wherein the holder is a channel-shaped member (19) having an inner bottom surface (22) defining said abutment surface and opposite inner side surfaces (21) from which projections (15, 15a) extend in opposite directions.

16. A holder according to any of the claims 10-15, wherein the holder (10) is frame-shaped and defines an opening (14) therein, the abutment surface (13) extending around and adjacent to said opening.

17. A holder according to any of the claims 10-16, wherein the holder has been integrally formed.

18. A testing device according to any of the claims 1-9, wherein the holder further comprises an upper side and a lower side in relation to an analysis instrument, and wherein the retaining means are positioned and shaped so as to allow insertion of the test member in to the holder from the upper side.

19. A holder according to any of the claims 10-17, wherein the holder further comprises an upper side and a lower side in relation to an analysis instrument, and wherein the retaining means are positioned and shaped so as to allow insertion of the test member in to the holder from the upper side.

20. A system comprising a cartridge for receiving, storing and unloading a plurality of stacked testing devices according to any of claims 1-9, and a plurality of stacked testing devices according to any of claims 1-9, wherein the cartridge comprises:
- a housing defining an internal passage for said stack of testing devices, said housing comprising:
- a lower charge opening for receiving said stack of testing devices,
- a moveable support member for supporting a lower testing device in said stack,
- an upper abutment surface for engaging with an upper testing device in the stack,
- an external protrusion comprising an abutment surface for abutting a support surface in a storage carousel in an analysis instrument so that the testing devices can be selective discharged for being processed in the analysis instrument, and
- an upper discharge opening, substantially aligned with said upper testing device, so as to allow discharge of said upper testing device by displacing the same along said abutment surface.

21. A system according to claim 20, wherein the housing of said cartridge is assembled by two halves, together defining oppositely side surfaces, and a front and a back surface.

22. A system according to claim 21, wherein the two halves of said cartridge are detachably or non-detachably assembled.

23. A system according to claims 20-22, wherein at least the discharge opening of said cartridge comprises guiding trails or incisions for guiding a testing device upon discharging.

24. A system according to claims 20-23, wherein the side surfaces of said cartridge comprise guiding trails for guiding said stack of testing devices through the passage.

25. A system according to claims 21-23, wherein the side surfaces of said cartridge further comprise at least one serrated track on the inside, forming one side of an internal one-way stair for a support member.

26. A system according to claim 25, said cartridge further comprising one-way means associated with the movable support member allowing the movable support member to move in a direction towards the upper abutment surface only.

27. A system according to claim 26, wherein said one-way means of said cartridge comprise at least one succession of teeth, such as a rack or ratchet teeth, and at least one pawl member co-operating therewith.

28. A system according to claims 25-27, wherein said cartridge comprises at least two pawl members, which are connected to the supporting member for co-operating with a succession of teeth formed on an inner side surface of the storage container, the free ends of the pawl members being spaced in the longitudinal direction of the container by a distance being different from a multiple of the pitch of the succession of teeth, preferably smaller than said pitch.

29. A system according to any of the claims 21-28, wherein at least one of the side surfaces of said cartridge further comprises a locking device in the vicinity of the discharge opening for preventing unintentional discharging of testing devices.

30. A system according to claim 29, wherein the locking device comprises at least one flexible protrusion obstructing at least a part of said discharge opening.

31. A system according to any of claims 20-30, further comprising a load device for loading a stack of testing devices according to any of claims 1-9 into said cartridge, the load device comprising:
- a base member,
- a first and a second column oppositely arranged and extending upwards from said base member, and being adapted to receive and hold one or more testing devices there between, and
- a lifting device for slidably lifting one of more testing devices along said columns.

32. A system according to claim 31, wherein each column of said load device comprises a groove for receiving and guiding an end of a test member.

33. A system according to claims 31-32, wherein the lifting device comprises a handle for manually sliding said lifting device along said columns.

34. A system according to claims 31-32, wherein the lifting device is automatically slid along said columns.

35. A system according to claims 31-34, wherein the lifting device further comprises a support surface for supporting at least a part of the lower testing device in said stack of testing devices.

36. A system according to claim 35, wherein the stack of testing devices is loaded into said cartridge and wherein the support member of said cartridge is arranged between said support surface of said lifting device and the lower most testing device in said stack.

37. A system according to claims 31-33, wherein the lifting device further comprises guiding means abutting a side portion of said columns so as to guide the device along the columns.

## Patentansprüche

1. Prüfvorrichtung zum Prüfen oder Analysieren von Fluids und umfassend
mindestens ein blech- oder plattenähnliches Prüfelement (11, 20) mit Analysereagenz und gegenüberliegenden Seitenflächen, die von Kantabschnitten umgeben sind, und
einen getrennt hergestellten Halter (10,19) mit Festhaltemitteln zum Aufnehmen und Festhalten des Prüfelements in einer vorbestimmten relativen Position im Halter,
wobei
das Festhaltemittel eine Anlagefläche (13, 22) zum Eingriff mit einer der Seitenflächen des Prüfelements und Vorsprünge (15, 15a) umfasst, die derart angeordnet und geformt sind, dass sie das Einführen des Prüfelements in den Halter durch Bewegen des Prüfelements in Eingriff mit der Anlagefläche ermöglichen, während sie in gegenüberliegende Kantenabschnitte des Prüfelements eingreifen,
und wobei
der Halter eine obere und eine untere komplementäre Fläche aufweist, um das Übereinanderstapeln einer Mehrzahl von Prüfvorrichtungen zu ermöglichen, **dadurch gekennzeichnet, dass** die komplementären Flächen derart geformt sind, dass sie das Versetzen von gestapelten Prüfvorrichtungen entlang einer Bewegungsbahn entlang einer Achse quer zu einer Längsachse des Stapels führen.

2. Prüfvorrichtung nach Anspruch 1, wobei die Vorsprünge (15) zahnförmig mit spitzen Enden sind.

3. Prüfvorrichtung nach Anspruch 1 oder 2, wobei jeder von mindestens einigen der Vorsprünge (15) eine Vorderkante (16) aufweist, die eine Rampe bildet, die zu einer Ebene abfällt, welche durch die Anlagefläche festgelegt ist, um das Einführen der Prüfelemente in den Halter zu erleichtern.

4. Prüfvorrichtung nach einem der vorhergehenden Ansprüche, wobei jeder von mindestens einigen der Vorsprünge (15) eine Hinterkante oder hintere Fläche (17) aufweist, die sich im Wesentlichen parallel und beabstandet zu einer Ebene erstreckt, die durch die Anlagefläche festgelegt ist.

5. Prüfvorrichtung nach einem der vorhergehenden Ansprüche, wobei die Vorsprünge derart angeordnet sind, dass sie von der durch die Anlagefläche festgelegten Ebene unterschiedlich beabstandet sind.

6. Prüfvorrichtung nach einem der vorhergehenden Ansprüche, wobei der Halter (19) ein kanalförmiges Element mit einer inneren Bodenfläche (22), welche die Anlagefläche festlegt, und gegenüberliegenden inneren Seitenflächen (21) ist, von denen sich Vorsprünge (15) in die gegenüberliegende Richtung erstrecken.

7. Prüfvorrichtung nach Anspruch 6, wobei das Prüfelement (20) ein längliches Element vom Typ "Querflussstick" ist, in das das zu prüfende Fluid an einem Ende (24) des länglichen Prüfelements eingetragen wird.

8. Prüfvorrichtung nach einem der Ansprüche 1-5, wobei der Halter (10) rahmenförmig ist und eine Öffnung (14) darin festlegt, wobei sich die Anlagefläche (13) um die Öffnung und benachbart dazu erstreckt.

9. Prüfvorrichtung nach einem der Ansprüche 1-8 zur Verwendung bei der kolorimetrischen Prüfung von Milch.

10. Halter zur Verwendung in einer Prüfvorrichtung nach einem der Ansprüche 1-9, wobei der Halter (10, 19) Mittel zum Aufnehmen und Festhalten eines blech- oder plattenähnlichen Prüfelements (11, 20) mit gegenüberliegenden Seitenflächen, die von Kantabschnitten umgeben sind, in einer vorbestimmten relativen Position im Halter aufweist, wobei die Festhaltemittel eine Anlagefläche (13, 22) zum Eingriff mit einer der Seitenflächen des Prüfelements und Vorsprünge (15, 15a) umfasst, die derart angeordnet und geformt sind, dass sie das Einführen des Prüfelements in den Halter durch Bewegen des Prüfelements in Eingriff mit der Anlagefläche ermöglichen, während sie in gegenüberliegende Kantenabschnitte des Prüfelements eingreifen, wobei der Halter eine obere und eine untere komplementäre Fläche aufweist, um das Übereinanderstapeln einer Mehrzahl von Haltern zu ermöglichen, **dadurch gekennzeichnet, dass** die komplementären Flächen derart geformt sind, dass sie das Versetzen von gestapelten Haltern entlang einer Bewegungsbahn entlang einer Achse quer zu einer Längsachse des Stapels führen.

11. Halter nach Anspruch 10, wobei die Vorsprünge (15) zahnförmig mit spitzen Enden sind.

12. Halter nach Anspruch 10 oder 11, wobei jeder von mindestens einigen der Vorsprünge eine Vorderkante (16) aufweist, die eine Rampe bildet, die zu einer Ebene abfällt, welche durch die Anlagefläche festgelegt ist, um das Einführen der Prüfelemente in den Halter zu erleichtern.

13. Halter nach einem der Ansprüche 10-12, wobei jeder von mindestens einigen der Vorsprünge eine Hinterkante oder hintere Fläche (17) aufweist, die sich im Wesentlichen parallel und beabstandet zu einer Ebene erstreckt, die durch die Anlagefläche festgelegt ist.

14. Halter nach einem der Ansprüche 10-13, wobei die Vorsprünge derart angeordnet sind, dass sie von der durch die Anlagefläche festgelegten Ebene unterschiedlich beabstandet sind.

15. Halter nach einem der Ansprüche 10-14, wobei der Halter ein kanalförmiges Element (19) mit einer inneren Bodenfläche (22), welche die Anlagefläche festlegt, und gegenüberliegenden inneren Seitenflächen (21) ist, von denen sich Vorsprünge (15, 15a) in die gegenüberliegende Richtung erstrecken.

16. Halter nach einem der Ansprüche 10-15, wobei der Halter (10) rahmenförmig ist und eine Öffnung (14) darin festlegt, wobei sich die Anlagefläche (13) um die Öffnung und benachbart dazu erstreckt.

17. Halter nach einem der Ansprüche 10-16, wobei der Halter einstückig ausgebildet ist.

18. Prüfvorrichtung nach einem der Ansprüche 1-9, wobei der Halter weiterhin eine Oberseite und eine Unterseite in Bezug auf ein Analyseinstrument umfasst und wobei die Festhaltemittel derart angeordnet und geformt sind, dass sie das Einführen des Prüfelements in den Halter von der Oberseite aus ermöglichen.

19. Halter nach einem der Ansprüche 10-17, wobei der Halter weiterhin eine Oberseite und eine Unterseite in Bezug auf ein Analyseinstrument umfasst und wobei die Festhaltemittel derart angeordnet und geformt sind, dass sie das Einführen des Prüfelements in den Halter von der Oberseite aus ermöglichen.

20. System, umfassend eine Patrone zur Aufnahme, Aufbewahrung und Abgabe einer Mehrzahl von gestapelten Prüfvorrichtungen nach einem der Ansprüche 1-9 sowie eine Mehrzahl von gestapelten Prüfvorrichtungen nach einem der Ansprüche 1-9, wobei die Patrone umfasst:
- ein Gehäuse, das einen Durchgang für den Stapel Prüfvorrichtungen im Inneren festlegt, wobei das Gehäuse umfasst:
- eine untere Beschickungsöffnung zur Aufnahme der Stapel Prüfvorrichtungen,
- ein bewegliches Trägerelement zum Tragen einer unteren Prüfvorrichtung in dem Stapel,
- eine obere Anlagefläche zum Eingriff mit einer oberen Prüfvorrichtung in dem Stapel,
- einen externen Vorsprung, umfassend eine Anlagefläche zum Anliegen an einer Trägerfläche in einem Aufbewahrungskarussell eines Analyseinstruments, sodass die Prüfvorrichtungen selektiv für die Bearbeitung in dem Analyseinstrument abgegeben werden können, und
- eine obere Abgabeöffnung, die im Wesentlichen an der oberen Prüfvorrichtung ausgerichtet ist, um das Abgeben der oberen Prüfvorrichtung durch Verschieben derselben entlang der Anlagefläche zu ermöglichen.

21. System nach Anspruch 20, wobei das Gehäuse der Patrone durch zwei Hälften, die gemeinsam gegenüberliegende Seitenflächen festlegen, sowie eine Vorder- und eine Hinterfläche zusammengesetzt ist.

22. System nach Anspruch 21, wobei die zwei Hälften der Patrone lösbar oder nicht lösbar zusammengesetzt sind.

23. System nach Anspruch 20-22, wobei mindestens die Abgabeöffnung der Patrone Führungskanten oder Einschnitte zum Führen einer Prüfvorrichtung nach der Abgabe umfasst.

24. System nach Anspruch 20-23, wobei die Seitenflächen der Patrone Führungskanten zum Führen des Stapels Prüfvorrichtungen durch den Durchgang umfassen.

25. System nach Anspruch 21-23, wobei die Seitenflächen der Patrone weiterhin mindestens eine gezahnte Spur im Inneren umfassen, die eine Seite einer Einwegtreppe für ein Trägerelement im Inneren ausbildet.

26. System nach Anspruch 25, wobei die Patrone weiterhin Einwegmittel umfasst, die mit dem beweglichen Trägerelement assoziiert sind und die Bewegung des beweglichen Trägerelements ausschließlich in eine Richtung zur oberen Anlagefläche ermöglichen.

27. System nach Anspruch 26, wobei die Einwegmittel der Patrone mindestens eine Zahnfolge umfassen, wie eine Zahnstange oder Knarrenzähne, und mindestens ein Klinkenelement, das damit zusammenwirkt.

28. System nach Anspruch 25-27, wobei die Patrone mindestens zwei mit dem Trägerelement verbundene Klinkenelemente zum Zusammenwirken mit einer an der Innenseitenfläche des Aufbewahrungsbehälters ausgebildeten Zahnfolge umfasst, wobei die freien Enden der Klinkenelemente in Längsrichtung des Behälters mit einem Abstand beanstandet sind, der nicht gleich eines Vielfachen der Teilung der Zahnfolge, vorzugsweise kleiner als die Teilung, ist.

29. System nach einem der Ansprüche 21-28, wobei mindestens eine der Seitenflächen der Patrone weiterhin eine Sperrvorrichtung in der Nähe der Abgabeöffnung zum Verhindern einer unbeabsichtigten Abgabe von Prüfvorrichtungen umfasst.

30. System nach Anspruch 29, wobei die Sperrvorrichtung mindestens einen flexiblen Vorsprung umfasst, der wenigstens einen Teil der Abgabeöffnung blockiert.

31. System nach einem der Ansprüche 20-30, weiterhin umfassend eine Beschickungsvorrichtung zum Beschicken der Patrone mit einem Stapel Prüfvorrichtungen nach einem der Ansprüche 1-9, wobei die Beschickungsvorrichtung umfasst:
- ein Sockelelement,
- eine erste und eine zweite einander gegenüberliegend angeordnete und sich vom Sockelelement nach oben erstreckende Säule, die dazu beschaffen sind, eine oder mehrere Prüfvorrichtungen dazwischen aufzunehmen und festzuhalten, und
- eine Hebevorrichtung zum schiebenden Anheben einer oder mehrerer Prüfvorrichtungen entlang der Säulen.

32. System nach Anspruch 31, wobei jede Säule der Beschickungsvorrichtung eine Nut zur Aufnahme und Führung eines Endes eines Prüfelements umfasst.

33. System nach Anspruch 31-32, wobei die Hebevorrichtung einen Griff für das manuelle Schieben der Hebevorrichtung entlang der Säulen umfasst.

34. System nach Anspruch 31-32, wobei die Hebevorrichtung automatisch entlang der Säulen geschoben wird.

35. System nach Anspruch 31-34, wobei die Hebevorrichtung weiterhin eine Trägerfläche zum Tragen mindestens eines Teils der unteren Prüfvorrichtung in dem Stapel Prüfvorrichtungen umfasst.

36. System nach Anspruch 35, wobei der Stapel Prüfvorrichtungen in die Patrone geladen wird und wobei das Trägerelement der Patrone zwischen der Trägerfläche der Hebevorrichtung und der untersten Prüfvorrichtung des Stapels angeordnet ist.

37. System nach Anspruch 31-33, wobei die Hebevorrichtung weiterhin Führungsmittel umfasst, die an einem Seitenabschnitt der Säulen anliegen, um die Vorrichtung entlang der Säulen zu führen.

## Revendications

1. Dispositif d'essai pour l'essai ou l'analyse de fluides et comprenant
au moins un élément d'essai (11, 20) de type feuille ou plaque incluant un réactif d'analyse et possédant des surfaces latérales opposées entourées de portions de bordure, et
un support (10, 19) produit séparément possédant un moyen de retenue pour recevoir et retenir l'élément d'essai dans une position relative prédéterminée dans le support,
dans lequel
ledit moyen de retenue comprend une surface de butée (13, 22) s'engageant avec l'une desdites surfaces latérales de l'élément d'essai et des saillies (15, 15a), qui sont positionnées et formées pour permettre l'insertion de l'élément d'essai dans le support en déplaçant l'élément d'essai pour l'engager avec ladite surface de butée tout en s'engageant avec les portions de bordure opposées de l'élément d'essai,
et dans lequel
ledit support possède des surfaces complémentaires supérieure et inférieure de manière à permettre l'empilement de plusieurs dispositifs d'essai les uns sur les autres, **caractérisé en ce que** lesdites surfaces complémentaires sont formées de manière à guider le déplacement mutuel des dispositifs d'essai empilés le long d'un trajet de mouvement le long d'un axe transversalement à un axe longitudinal de l'empilement.

2. Dispositif d'essai selon la revendication 1, dans lequel les saillies (15) sont en forme de dent avec des extrémités pointues.

3. Dispositif d'essai selon les revendications 1 ou 2, dans lequel chacune d'au moins certaines des saillies (15) possède un bord antérieur (16) formant une rampe inclinée vers un plan défini par la surface de butée de manière à faciliter l'insertion de l'élément d'essai dans le support.

4. Dispositif d'essai selon l'une quelconque des revendications précédentes, dans lequel chacune d'au moins certaines des saillies (15) possède un bord ou une surface postérieur(e) (17) s'étendant essentiellement parallèlement à et espacé(e) d'un plan défini par la surface de butée.

5. Dispositif d'essai selon l'une quelconque des revendications précédentes, dans lequel les saillies sont positionnées de manière à être espacées différemment par rapport au plan défini par la surface de butée.

6. Dispositif d'essai selon l'une quelconque des revendications précédentes, dans lequel le support (19) est un élément en forme de canal possédant une surface inférieure interne (22) définissant ladite surface de butée et des surfaces latérales internes (21) opposées depuis lesquelles des saillies (15) s'étendent dans des directions opposées.

7. Dispositif d'essai selon la revendication 6, dans lequel l'élément d'essai (20) est un élément allongé de type « bâtonnet à écoulement latéral », dans lequel le fluide à tester est fourni à une extrémité (24) de l'élément d'essai allongé.

8. Dispositif d'essai selon l'une quelconque des revendications 1 à 5, dans lequel le support (10) est en forme de cadre et définit une ouverture (14) dans celui-ci, la surface de butée (13) s'étendant autour et à côté de ladite ouverture.

9. Dispositif d'essai selon l'une quelconque des revendications 1 à 8 destiné à être utilisé pour le dosage colorimétrique du lait.

10. Support destiné à être utilisé dans un dispositif d'essai selon l'une quelconque des revendications 1 à 9, ledit support (10, 19) comprenant des moyens pour recevoir et retenir un élément d'essai (11, 20) de type feuille ou plaque, qui possède des surfaces latérales opposées entourées de portions de bordure, dans une position relative prédéterminée dans le support, lesdits moyens de retenue comprenant une surface de butée (13, 22) pour s'engager avec l'une desdites surfaces latérales de l'élément d'essai et des saillies (15, 15a), qui sont positionnées et formées pour permettre l'insertion de l'élément d'essai dans le support en déplaçant l'élément d'essai pour l'engager avec ladite surface de butée tout en s'engageant avec les portions de bordure opposées du support,
dans lequel le support possède des surfaces complémentaires supérieure et inférieure de manière à permettre l'empilement de plusieurs supports les uns sur les autres, **caractérisé en ce que** lesdites surfaces complémentaires sont formées de manière à guider le déplacement mutuel des supports empilés le long d'un trajet de mouvement le long d'un axe transversalement à l'axe longitudinal de l'empilement.

11. Support selon la revendication 10, dans lequel les saillies (15) sont en forme de dent avec des extrémités pointues.

12. Support selon les revendications 10 ou 11, dans lequel chacune d'au moins certaines des saillies possède un bord antérieur (16) formant une rampe inclinée vers un plan défini par la surface de butée de manière à faciliter l'insertion de l'élément d'essai dans le support.

13. Support selon l'une quelconque des revendications 10 à 12, dans lequel chacune d'au moins certaines des saillies possède un bord ou une surface postérieur(e) (17) s'étendant essentiellement parallèlement à et espacé(e) d'un plan défini par la surface de butée.

14. Support selon l'une quelconque des revendications 10 à 13, dans lequel les saillies sont positionnées de manière à être espacées différemment par rapport au plan défini par la surface de butée.

15. Support selon l'une quelconque des revendications 10 à 14, dans lequel le support est un élément en forme de canal (19) possédant une surface inférieure interne (22) définissant ladite surface de butée et des surfaces latérales internes (21) opposées depuis lesquelles des saillies (15, 15a) s'étendent dans des directions opposées.

16. Support selon l'une quelconque des revendications 10 à 15, dans lequel le support (10) est en forme de cadre et définit une ouverture (14) dans celui-ci, la surface de butée (13) s'étendant autour et à côté de ladite ouverture.

17. Support selon l'une quelconque des revendications 10 à 16, dans lequel le support a été formé d'une seule pièce.

18. Dispositif d'essai selon l'une quelconque des revendications 1 à 9, dans lequel le support comprend en outre un côté supérieur et un côté inférieur par rapport à un instrument d'analyse, et dans lequel les moyens de retenue sont positionnés et formés pour permettre l'insertion de l'élément d'essai dans le support depuis le côté supérieur.

19. Support selon l'une quelconque des revendications 10 à 17, dans lequel le support comprend en outre un côté supérieur et un côté inférieur par rapport à un instrument d'analyse, et dans lequel les moyens de retenue sont positionnés et formés pour permettre l'insertion de l'élément d'essai dans le support depuis le côté supérieur.

20. Système comprenant une cartouche destinée à recevoir, stocker et décharger plusieurs dispositifs d'essai empilés selon l'une quelconque des revendications 1 à 9, et plusieurs dispositifs d'essai empilés selon l'une quelconque des revendications 1 à 9, dans lequel la cartouche comprend :
- un corps définissant un passage interne pour ledit empilement de dispositifs d'essai, ledit corps comprenant :
- une ouverture de chargement inférieure destinée à recevoir ledit empilement de dispositifs d'essai,
- un élément de soutien mobile destiné à soutenir un dispositif d'essai inférieur dans ledit empilement,
- une surface de butée supérieure destinée à s'engager avec un dispositif d'essai supérieur dans l'empilement,
- une protubérance externe comprenant une surface de butée pour buter contre une surface de soutien dans un carrousel de stockage d'un instrument d'analyse de manière à ce que les dispositifs d'essai puissent être déchargés de façon sélective pour être traités dans l'instrument d'analyse, et
- une ouverture de déchargement supérieure, essentiellement alignée avec ledit dispositif d'essai supérieur, de manière à permettre le déchargement dudit dispositif d'essai supérieur en déplaçant celui-ci le long de ladite surface de butée.

21. Système selon la revendication 20, dans lequel le corps de ladite cartouche est formé de deux moitiés assemblées, définissant ensemble des surfaces latérales opposées, et une surface avant et une surface arrière.

22. Système selon la revendication 21, dans lequel les deux moitiés de ladite cartouche sont assemblées de manière détachable ou non détachable.

23. Système selon les revendications 20 à 22, dans lequel au moins l'ouverture de déchargement de ladite cartouche comprend des voies ou incisions de guidage destinées à guider un dispositif d'essai lors du déchargement.

24. Système selon les revendications 20 à 23, dans lequel les surfaces latérales de ladite cartouche comprennent des voies de guidage destinées à guider ledit empilement de dispositifs d'essai au travers du passage.

25. Système selon les revendications 21 à 23, dans lequel les surfaces latérales de ladite cartouche comprennent en outre au moins une glissière crantée sur l'intérieur, formant un côté d'un « escalier » interne à sens unique pour un élément de soutien.

26. Système selon la revendication 25, ladite cartouche comprenant en outre des moyens à sens unique associés à l'élément de soutien mobile permettant à l'élément de soutien mobile de se déplacer dans une direction uniquement vers la surface de butée supérieure.

27. Système selon la revendication 26, dans lequel lesdits moyens à sens unique de ladite cartouche comprennent au moins une succession de dents, telles que des dents de crémaillère ou triangulaires, et au moins un élément cliquet fonctionnant avec celles-ci.

28. Système selon les revendications 25 à 27, dans lequel ladite cartouche comprend au moins deux éléments cliquet, qui sont reliés à l'élément de soutien pour fonctionner avec une succession de dents formées sur une surface latérale interne du récipient de stockage, les extrémités libres des éléments cliquet étant espacées dans la direction longitudinale du récipient d'une distance qui est différente d'un multiple du pas de la succession de dents, de préférence inférieure au dit pas.

29. Système selon l'une quelconque des revendications 21 à 28, dans lequel au moins une des surfaces latérales de ladite cartouche comprend en outre un dispositif de verrouillage à proximité de l'ouverture de déchargement destiné à empêcher le déchargement involontaire des dispositifs d'essai.

30. Système selon la revendication 29, dans lequel le dispositif de verrouillage comprend au moins une protubérance souple obstruant au moins une partie de ladite ouverture de déchargement.

31. Système selon l'une quelconque des revendications 20 à 30, comprenant en outre un dispositif de chargement pour charger dans ladite cartouche un empilement de dispositifs d'essai selon l'une quelconque des revendications 1 à 9, le dispositif de chargement comprenant :
- un élément de base,
- une première et une deuxième colonne disposées à l'opposé l'une de l'autre et s'étendant vers le haut depuis ledit élément de base, et étant adaptées pour recevoir et contenir entre elles un ou plusieurs dispositifs d'essai, et
- un dispositif de soulèvement pour soulever par coulissement l'un de plusieurs dispositifs d'essai le long desdites colonnes.

32. Système selon la revendication 31, dans lequel chaque colonne dudit dispositif de chargement comprend une rainure destinée à recevoir et guider une extrémité d'un élément d'essai.

33. Système selon les revendications 31 à 32, dans lequel le dispositif de soulèvement comprend une poignée pour faire coulisser manuellement ledit dispositif de soulèvement le long desdites colonnes.

34. Système selon les revendications 31 à 32, dans lequel le dispositif de soulèvement coulisse automatiquement le long desdites colonnes.

35. Système selon les revendications 31 à 34, dans lequel le dispositif de soulèvement comprend en outre une surface de soutien destinée à soutenir au moins une partie du dispositif d'essai inférieur dans ledit empilement de dispositifs d'essai.

36. Système selon la revendication 35, dans lequel l'empilement de dispositifs d'essai est chargé dans ladite cartouche et dans lequel l'élément de soutien de ladite cartouche est disposé entre ladite surface de soutien dudit dispositif de soulèvement et le dispositif d'essai situé le plus bas dans ledit empilement.

37. Système selon les revendications 31 à 33, dans lequel le dispositif de soulèvement comprend en outre des moyens de guidage venant buter contre une portion latérale desdites colonnes de manière à guider le dispositif le long des colonnes.
